# EUROPEAN PATENT SPECIFICATION

(11) **EP 2 240 167 B1**
(45) Date of publication and mention of the grant of the patent: **13.11.2013**
(21) Application number: 08864457.0
(22) Date of filing: 18.12.2008
(51) Int. Cl.: A61K 31/00, A61K 47/12, A61K 47/24, A61K 47/44, A61K 9/00

(54) **EXTRUDED ROD-SHAPED DEVICES FOR CONTROLLED RELEASE OF BIOLOGICAL SUBSTANCES IN HUMANS AND ANIMALS**
EXTRUDIERTE STANGENFÖRMIGE VORRICHTUNGEN ZUR KONTROLLIERTEN FREISETZUNG VON BIOLOGISCHEN SUBSTANZEN BEI MENSCHEN ODER TIEREN
DISPOSITIFS EXTRUDÉS EN FORME DE BAGUETTE POUR LA LIBÉRATION CONTROLÉE DE SUBSTANCES BIOLOGIQUES CHEZ L'HOMME ET L'ANIMAL

(30) Priority: 21.12.2007 EP 07123958
(43) Date of publication of application: 20.10.2010
(73) Proprietor: Ludwig-Maximilians-Universität, 80539 München (DE)
(72) Inventor: WINTER, Gerhard, 82377 Penzberg (DE); SCHULZE, Sandra, 80639 München (DE)
(74) Representative: Neuefeind, Regina
(86) International application number: PCT/EP2008/010796
(87) International publication number: WO 2009/080275

(56) References cited:
- WO-A-02/00137
- WO-A-2004/011054
- WO-A-2005/102284
- WO-A-2007/001451
- US-A- 4 450 150
- US-A1- 2005 208 122
- US-A1- 2006 159 721

## Description

### Field of the invention

The present invention relates to an extruded rod-shaped device which comprises at least one biological substance and a lipoid composition that comprises a high melting lipid or lipoid component and a low melting lipid or lipoid component. The extruded rod-shaped device according to the present invention is obtainable by extrusion of a preparation comprising the lipoid composition and the at least one biological substance, the preparation being extruded at a temperature which is at or above the melting point of the low melting lipid or lipoid component but below the melting point of the high melting lipid or lipoid component. Such an extruded rod-shaped device is capable of continuously and homogenously releasing the biological substance into the body of an animal or a human while maintaining the biological activity the biological substance and may for example be used as an implant.

### Background of the invention

In comparison to low molecular weight drugs, the higher selectivity of biological substances such as proteins, polypeptides, nucleic acids or virus-like particles or the like often allows a better treatment of serious, life-threatening, and chronic diseases such as cancer, rheumatoid arthritis, hepatitis and others. However, due to their fragile, three-dimensional macromolecular structure, biological substances are often susceptible to a variety of chemical or physical degradation pathways and mostly require parenteral administration for systemic delivery. Hence, the development of suitable formulations on which the native structure and the activity of biological substances is maintained during preparation, delivery, shipping and long-term storage has become one of the most challenging tasks.

Because of the short half-lives of sensitive biological substances after parenteral administration, the parenteral application by injection or infusion of e.g. protein and peptide drugs in solution is not practicable in every case nor it is convenient to the majority of the patients. There was thus a strong need in the technical field to develop alternative parenteral routes of administration of biological substances.

To overcome the problem of many repeated injections, potentially applied in rather short intervals, which is associated with poor patient compliance, side effects, or cost consuming hospitalisation, the idea of parenteral depots was born. Such depots may be delivered into the body at various sites with the aim to release the biological substances over an extended period of time. Many application sites for delivery and manifold release time programmes were considered, depending on the indication to be treated, the pharmacokinetics of the drug, its dose and many other factors. So, a variety of parenteral depot systems were investigated and described in the prior art.

Several systems, like microspheres, liposomes, as well as solid and *in-situ* forming implants, have been suggested as delivery technologies for biological substances [Gombotz, W. R. and Pettit, D. K., Biodegradable polymers for protein and peptide drug delivery, Bioconjug Chem. 6: 332-351 (1995); Schwendeman, S. P., Recent advances in the stabilization of proteins encapsulated in injectable PLGA delivery systems, Critical Reviews in Therapeutic Drug Carrier Systems. 19: 73-98 (2002); van de, Weert M., Hennink, W. E., and Jiskoot, W., Protein instability in poly(lacticco-glycolic acid) microparticles, Pharmaceutical research. 17: 1159-1167 (2000).]. Among the plethora of investigated synthetic and natural polymers, biodegradable poly(α-hydroxy esters) and in particular poly(lactic acid), PLA, and poly(lactic-co-glycolic acid), PLGA, found most widespread use. Such polymer systems were developed, due to the fact, that the variety of imaginable polymers allows theoretically infinite variations in solubility and hydrophobicity, mechanical properties, diffusivity and other factors. However, the only PLGA-protein drug product was withdrawn from the market few years after launch, due to apparent loss of success, i.e. acceptance by the patients, doctors and due to high manufacturing costs [Genentech press release, Jun 1 2004. web 4-6-0004. Ref Type: Electronic Citation].

The parameters defining the acceptance and safety of parenteral depot systems are excellent applicability and tolerability (painlessness during and after application), lack of toxicity, biodegradability, stability of drug and product, sufficiently long retardation or in other words release kinetics that fit well to the desired dosing schedule, and release of pure, non degraded biological substance. Apparently, the parenteral depot systems developed so far did not meet the criteria set out by the regulatory bodies and/or the market expectations. A further set of criteria that is important for the manufacturer of such systems is the manufacturing costs, the upscalability of the production processes to commercial size and the versatility of the technology platform to be applicable for a wide variety of uses.

As the restrictions of polymer based parenteral depot systems became obvious, another group of depot systems based on lipid materials was investigated. Lipids are excellently biocompatible, what has been shown in animal studies. Due to their natural origin, they are non-toxic and degradable into fatty acids, glycerol, alcohols etc. and finally metabolized by the physiological metabolic cycles of mammals. Furthermore, lipids are by definition rather hydrophobic and the diffusion of water into lipid systems is generally low. In contrast to polymer systems (like PLGA) lipid systems therefore show different swelling, degradation and release behaviour, which is advantageous for protein drugs and their release. In general, lipid systems are non swellable or swellable only to a small amount due to their inherent lipophilicity. Furthermore, lipids are available in a rather broad range of physicochemical properties and thereby allow the adjustment of formulation properties, once a system platform has been established. Lipids can be molten and formed under moderate temperature conditions, what may allow the production of protein loaded systems without much negative temperature effects on such drugs. In a specific setup of such production processes lipids can be processed without organic solvents when producing pharmaceutical carrier forms, which is of importance considering the sensitivity of certain biological substances to organic solvents.

Although lipid systems appear beneficial over other alternatives the use of such systems according to the state of the art leaves still important drawbacks and unresolved problems open. The concept still remains mainly in the research phase and has not reached the market or near market clinical studies for the treatment of human diseases. Release kinetics are often not yet convincing, some manufacturing processes are complicated and therefore not really cost effective nor up-scalable, some other processes still use organic solvents too. The stability of certain biological substances, such as proteins, in certain formulations is insufficient and in many cases no stability data are available at all.

The parenteral depot systems for biological substances which are based on lipids can be divided into solid carrier forms such as microparticles, solid implants, oily systems and lipid bilayer based systems such as liposomes and cubic phase gels.

Microparticles provide the chance to apply lipid based depots systems via an aqueous suspension that may be reconstituted immediately before application by suspending the preformed dry lipid particles in an aqueous reconstitution fluid. Such suspensions may have the virtual advantage that they provide convenience by the application through a thin, large Gauge needle. This implies the need to control and limit the size and size distribution of the microparticles in suspension very closely in the range of micrometers. Furthermore, microparticles basically for geometric reasons provide an overall less sustained release pattern compared to solid carrier forms due to the fact that surface to volume ratio and diffusion pathway are of course much more unfavourable in microparticles than in solid carrier forms sized in the mm range instead of the micrometer range. Furthermore, the versatility of shape and sizes of solid carrier forms permits the adjustment of drug release rates and they, when implanted, inhere the possibility of a surgical removal, if adverse events necessitate an interruption of the therapy.

Hence, from the many potentially applicable lipid based depot systems, the solid carrier form, typically described as solid implants, has many advantages over other lipid based systems. Solid carrier forms can be imagined in any size and shape and have the advantage of generally high storage stability, especially compared to liquid carrier forms, i.e. suspensions of biological substances in lipids.

Several formulations and processes have been published describing lipid based depot systems. Solid lipidic implant systems are mostly prepared by compression of lipid blends. The high compressibility of lipids allows the formation of solid matrices by traditional compression at mild conditions. Thus, several authors used this technique to demonstrate the suitability of lipids as carriers for pharmaceutical substances. However, all these methods were based on a manufacturing method in lab-scale with the help of self-made equipment. So far, no attempts have been made to prepare lipid-based delivery systems for biological substances with up-scalable techniques, such as extrusion.

In the following some important references in the field of the art shall be named and commented. However, all systems proposed do not fulfil the expectations on stable, parenterally applicable lipid based solid carrier forms with good sustained release properties for biological substances and a cheap, easy to use and up-scalable production process.

WO 03/049719 A2 describes carrier systems for stabilizing and controlled release of active substances, the carrier system being produced by completely melting lipids, dispersing active substances into such melts and extruding the composition. However, the required temperatures necessary for the production of such carrier systems are highly undesirable with respect to both stability of biological substances and re-crystallisation of the lipid matrix after cooling. The latter may occur in an uncontrollable manner leading to undesirable modifications of the lipids and thereby insufficiently controlled release.

EP 0 523 330 A1 relates to an implantable device for the parenteral administration of an essentially uniform and continuous amount of biologically active protein, peptide or polypeptide over an extended period of time. However, only a specifically formed, layered implant is described, wherein an indentation acts as the passageway for the release of the drug. Such a special geometric form is difficult and expensive to produce and small deviations from the desired form would lead to large deviations from the necessary release kinetics. Furthermore, it is suggested producing the implants by spray drying, which would require the loading of an organic polymer with the biological substance.

US 5,750,100 relates to parenteral pharmaceutical preparations for proteins comprising a polyglycerol diester of a saturated fatty acid having about 16 to 30 carbon atoms. However, such lipid derivatives are undesirable for their compromised lipophilicity, i.e. a higher degree of swelling and water uptake during the release within aqueous systems. Furthermore, it is suggested to dissolve the physiologically active polypeptide in the molten diester during the manufacturing procedure which is highly undesirable with respect to both stability of biological substances and re-crystallisation of the lipid matrix after cooling.

WO 2005/123138 A1 relates to a pharmaceutical composition in form of a pellet comprising pharmaceutically active substances, one or more lipids, one or more hydrophilic excipients and one or more water-insoluble binding agents, the pellet being prepared without melting the lipids. However, only oral use and close to linear release over less than 8 hours is achieved with such compositions. Parenteral application is not considered.

WO 00/01416 A1 relates to pharmaceutical compositions for oral administration comprising at least one active ingredient and a low-melting wax. However, only oral use of such compositions in the form of capsules is considered since an enhanced drug absorption is achieved by such means, i.e. the intention of this reference is not retardation but the opposite, fast, favourable release aiming at high oral bioavailability. Parenteral application is not considered.

WO 93/10758 A1 describes a composition for the sustained release of a biologically active therapeutic agent wherein the matrix of the sustained release composition is composed of an amorphous carbohydrate glass matrix comprising a suitable carbohydrate and an agent which retards the crystallization of the carbohydrate, and a biologically active therapeutic agent, such as biologically active polypeptides, antibiotics and vitamins, and a water-insoluble solid wax dispersed throughout the matrix. However, the required temperatures are highly undesirable with respect to substance stability after cooling. Furthermore, a different matrix composition is described that is no longer dominated by the properties of the lipids used.

US 5,380,535 relates to a non-aqueous, chewable composition for oral delivery of unpalatable drugs, the composition containing the drug intimately dispersed or dissolved in a lipid that is solid at room temperature. However, parenteral application is not considered.

US 4,483,847 is concerned with a process for the production of pharmaceutical compositions with a retarded liberation of active material. During the process, a powdered mixture is prepared of the active material, of the lipid components, as well as of conventional filling materials and disintegrating materials or swelling agents as liberation controlling components. After compressing the powdered mixture, the resulting mass is granulated and the granulates can be pressed to give tablets. Extruded rod-shaped devices for sustained delivery of biological substances are not considered.

WO2005102284 discloses an implant for sustained delivery, obtainable by coating a protein drug with a hydrophilic polymer, mixing the coated solid protein with a lipid and compressing or extruding the mixture. The lipid is solid at room temperature and is selected from fatty acids, monoglycerides, diglycerides, triglycerides, sorbitan fatty acid esters, phospholipids, sphingolipids, cholesterol, waxes. There is also a lipoid protein stabilizer selected from sugars, polyols, surfactants. WO2004011054 relates to a depot composition comprising a polymer matrix comprising a plurality of lipoid polymers and a beneficial agent such as protein. The plurality of polymers comprises a first polymer of a low molecular weight (LMW), a second polymer of a high molecular weight (HMW) polymer and a third polymer of a medium molecular weight (MMW) polymer.

WO0200137 describes a biodegradable vehicle or delivery system comprising a biodegradable lipoid polymer, a lipoid plasticizer such as glyceryl di and tri stearate, glyceryl palmitostearate and a biologically active substance such as protein.

Considering the limitations of conventional drug carriers such as liposomes, lipid emulsions, nanoparticles and microspheres as outlined above there is an obvious demand for an alternative carrier system for the controlled delivery of biological substances to circumvent the drawbacks of traditional systems particularly with regard to preparation, stability, toxicity and modification of biodistribution.

### Summary of the invention

The present invention introduces a new type of carrier system characterized as rod-shaped extruded devices comprising at least 50% per weight of a lipoid composition and at least one biological substance, the lipoid composition comprising a high melting lipid or lipoid component and a low melting lipid or lipoid component, which are extruded at a temperature, which is at or above the melting point of the low melting lipid or lipoid component but below the melting point of the high melting lipid or lipoid component in a screw type extruder. These carriers provide the possibility for the controlled delivery of biological substances such as protein drugs or other biological materials primarily by the parenteral route of administration.

The invention presented here fulfils the following requirements in an excellent manner whereas other carrier systems known from the literature lack at to meet at least one, mostly more than one of the parameters:
- Excellent biocompatibility of the extruded devices and device materials
- Little or no aggregation and disintegration of biological substances occurring during incorporation and storage in a lipid matrix
- Little or no aggregation and disintegration of biological substances during release from rod-shaped extruded devices according to the invention
- Sustained and continuous, preferably close to zero order release kinetics, with an acceptable low initial burst
- Duration of release in the range of more than one, preferably more than two weeks
- Easy, cheap, reproducible manufacturing process that can be upscaled into commercial dimensions
- Good mechanical properties, such as a high breaking strength, of the inventive devices that allow convenient handling and implantation
- Good storage stability
- Very homogeneous biological substance distribution throughout the extruded rod-shaped device

Furthermore, the preparation of extruded rod-shaped devices according to the invention can avoid the employment of any toxicologically active additives such as organic solvents or toxic monomers, and can be accomplished by easy-to-handle techniques, such as extrusion.

Extruded rod-shaped devices according to the invention can be used in the following fields of application:
a) as a parenteral delivery system with sustained biodistribution for biological substances;
b) as a delivery system for nasal, pulmonary, rectal, vaginal, dermal and/or buccal administration in humans and animals;
c) as a delivery system for subcutanous administration in humans and animals;
d) as a delivery system for use in agricultural applications or other *in vitro* release situations where biological substances shall be sustained released over time;
e) as a delivery system that allows the temperature-dependent modulate release of the biological substance at the site of action (temperature variations can be adjusted by a separate heat treatment or in combination with certain diseases (such as inflammatory reactions) or treatments (such as laser therapy)).

Basically the requirements are met by the following process and devices:

The present invention relates to an extruded rod-shaped device for sustained delivery of biological substances, obtainable by a process comprising
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the preparation of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

In a preferred embodiment of the invention, the lipoid composition is selected in a way that it is solid at room temperature. Preferably, the weight ratio between the high melting lipid or lipoid component and the low melting lipid or lipoid component in the lipoid composition is between 10:1 1 to 1:10, more preferably between 10:1 to 1:1, even more preferably between 8:1 to 2:1, and most preferably between 5:1 to 2:1.

In a preferred embodiment of the invention, the lipids or lipoid components are selected in a way that the melting point of the high melting lipid or lipoid component is at least 20°C, preferably at least 25°C, more preferably at least 30°C, and most preferably at least 35°C higher than the melting point of the low melting lipid or lipoid component. Preferably, the melting point of the high melting lipid or lipoid component is above 50°C, more preferably above 60°C, even more preferably above 65°C and most preferably above 70°C. Preferably, the melting point of the low melting lipid or lipoid component is below 50°C, more preferably below 45°C, even more preferably below 40°C and most preferably below 37°C. It is also preferred that the melting point of the low melting lipid or lipoid component is above 20°C.

Both the high melting lipid or lipoid component and the low melting lipid or lipoid component may e.g. be selected from the class of fatty acid mono-, di- and/or triglycerides, and salts and derivatives thereof.

The at least one biological substance is preferably selected from the group consisting of proteins, polypeptides, peptides and nucleic acids, and salts and derivatives thereof. The at least one biological substance may also be a virus-like particle, a virus, a protein aggregate or another kind of multimer.

Preferably, the device according to the invention comprises at least one excipient which modifies the release of the at least one biologically active substance from the implantable device and/or modifies the biodegradation of the lipids or lipoid components of the implantable device and/or stabilises the biological substance during manufacturing, storage and release. Furthermore, the excipient might modify the solubility of the biological substance or can itself feature slow dissolution behavior. The excipient can e.g. be a hydrophilic polymer, a sugar, a polyol, a surfactant and/or a water-soluble salt or any other excipient known in the art which has the above mentioned functions. In some embodiments of the invention, the excipient modifying the release of the at least one biologically active substance from the implantable device and/or modifying the biodegradation of the lipids or lipoid components of the implantable device can be selected from the group consisting of carboxymethylcellulose, gelantine and starch.

Preferably, the hydrophilic polymer is selected from the group consisting of polyethylene glycol, polyvinylpyrrolidone, polyvinylalcohol, dextran, dextran sulfate, chondroitin sulfate, dermatan sulfate, heparan sulfate, keratan sulfate, hyaluronic acid, chitosan, albumin, fibrin, cyclodextrin and mixtures thereof. In such an embodiment of the invention, the excipient can modify the release of the at least one biologically active substance from the extruded rod-shaped device by leaching out of the implantable device, thereby facilitating the formation of pores. Alternatively or additionally, the excipient may have a lipase activity, thereby modifying the biodegradation of the lipids or lipoid components comprised by the implantable device. Alternatively or additionally, a lipid excipient can be added that facilitates the biodegradation of the lipids or lipoid components comprised by the implantable device (i) by the formation of a mixed-lipid phase, (ii) by the formation of an eutectic phase, (iii) by its amphiphilic character, or by (iv) its high susceptibility to lipase cleavage. Such an excipient may be selected from the group consisting of mixed and mono-acid triglycerides, diglycerides, monoglycerides, fatty acids, and phospholipids.

In a preferred embodiment, the device according to the invention comprises a high melting lipid or lipoid component and a low melting lipid or lipoid component, both having a stable lipid modification after extrusion, the stable lipid modification more preferably being a beta modification.

Preferably, the device according to the invention has a diameter size of at least 0.1 mm and/or a length of at least 5 mm. Also preferably, the device according to the invention has a diameter size of at least 0.1 mm and a length to diameter ratio of at least more than. 1 to 1.5. Such a device may have a good mechanical stability, which allows handling, transport as well as administration, e.g. administration through a springe.

It is preferred that the at least one biological substance is delivered over a period of at least one week. More preferably the at least one biological substance is delivered over a period of at least two weeks, and even more preferably over a period of at least three weeks.

The present invention further relates to a method of producing a rod-shaped device for sustained delivery of a biological substance, comprising
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the composition of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

Preferably, said preparation further comprises at least one excipient which modifies the release of the at least one biologically active substance from the implantable device and/or modifies the biodegradation of the lipids or lipoid components of the implantable device and/or stabilises the biological substance during manufacturing, storage and release. Furthermore, the excipient might modify the solubility of the biological substance or can itself feature slow dissolution behavior.

Preferably, the excipient is selected from the group consisting of a hydrophilic polymer, a sugar, a polyol, a surfactant and/or a water-soluble salt. Also preferably, the hydrophilic polymer is selected from the group consisting of polyethylene glycol, polyvinylpyrrolidone, polyvinylalcohol, polyethyleneimine, dextran, dextran sulfate, chondroitin sulfate, dermatan sulfate, heparan sulfate, keratan sulfate, hyaluronic acid, chitosan, albumin, collagen, fibrin, cyclodextrin and mixtures thereof. In an embodiment of the invention, the excipient modifies the release of the at least one biologically active substance from the extruded rod-shaped device by leaching out of the implantable device, thereby facilitating the formation of pores. Alternatively or additionally, the excipient may have a lipase activity, thereby modifying the biodegradation of the lipids or lipoid components comprised by the implantable device. Alternatively or additionally, a lipid excipient can be added that facilitates the biodegradation of the lipids or lipoid components comprised by the implantable device (i) by the formation of a mixed-lipid phase, (ii) by the formation of an eutectic phase, (iii) by its amphiphilic character, or by (iv) its high susceptibility to lipase cleavage. Such an excipient may be selected from the group consisting of mixed and mono-acid triglycerides, diglycerides, monoglycerides, fatty acids, and phospholipids.

In a preferred embodiment of the invention, extrusion is carried out at a temperature which is at or 1°C to 25°C above the melting point of the low melting lipid or lipoid component, preferably between 1 °C and 20°C above the melting point of the low melting lipid or lipoid component, and more preferably between 1°C and 10°C above the melting point of the low melting lipid or lipoid component. Particularly preferably, extrusion is carried out at a temperature which is between 40°C and 60°C. Most preferably, extrusion is carried out at a temperature as low as possible.

Extrusion may be carried out in a continuous mode. Preferably, the extruder according to the invention is equipped with at least one pair of fully intermeshing, co-rotating, extruder elements. Such an extruder may be a twin screw extruder.

The extruded device may then be obtained from the extrudate by cutting or breaking-off the extrudate into small sections.

Preferably, the production process of the extruded rod-shaped device is a single-step process.

The present invention further relates to an extruded rod-shaped device according to the invention, or which is produced by a method according to the invention, for use as a parenteral delivery system with sustained delivery of biological substances. Preferably, such a device is for subcutaneous, nasal, pulmonary, rectal, dermal, buccal and/or vaginal administration. The extruded rod-shaped device preferably has an injectable size and may be inserted by injection or needle-free injection, but, if desired, may be placed at or within an administration site, or may be inserted at an administration site by surgical operation or by any other means.

Other objects, advantages, and features of the present invention will become apparent from the following specification.

### Brief description of the drawings

Figure 1: WAXS analyses of H12/Dynasan 118 extrudates directly after twin screw extrusion. All extrudate formulations revealed the typical short spacings of the stable beta modification at 0.46, 0.39 and 0.37 nm.

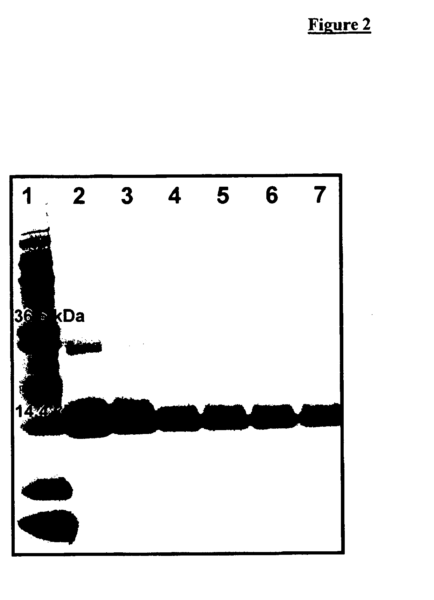

Figure 2: SDS-PAGE of IFN α-2a extracted from lipidic extrudates. After extrusion the protein was extracted and undergone SDS-PAGE with subsequent silver staining. Lane 1: Molecular weight standard, lane 2: IFN α-2a standard, lane 3: IFN α-2a after lyophilisation (marker), lane 4-7: IFN α-2a extracted from lipidic implants. The extrudates formulations were 80% H12/Dynasan 118, 10% lyophilised IFN α-2a and 10% PEG (lane 4 and lane 5 extrudates received at the beginning and at the end of the extrusion procedure, respectively) and 70% H 12/Dynasan 118, 10% lyophilised IFN α-2a and 20% PEG (lane 6 and lane 7 extrudates received at the beginning and at the end of the extrusion procedure, respectively). All samples revealed only the monomer and a marginal dimer fraction. In comparison to the protein standard and the lyophilised raw material the extrusion process induces no further aggregation or fragmentation.
**Figure 3****:** Second derivative KBr-pellet-transmission spectra of IFN-α-2a before extrusion and after extrusion with a lipidic blend based on H12 and Dynasan 118. The band at 1653 cm⁻¹ was largely unaffected by the extrusion procedure. Thus, extrusion of IFN α-2a embedded in a H12/Dynasan 118 blend comprising either 10% or 20% PEG does not induce important changes in the secondary protein structure.
**Figure 4****:** Effect of PEG content and implant diameter on the *in vitro* release behaviour of IFN α-2a from lipidic extrudates. For extrusion 10 % IFN α-2a/HP-β-CD lyophilisate was blended with 10% or 20% PEG and 80% or 70% H12/Dynasan 118. Extrusion was performed with a twin screw extruder at 40 °C. The diameter of the prepared rods was 0.5 mm (A), 1.0 mm (B), or 1.9 mm (C) (average +/- SD; n = 3). By changing the extrudate diameter, protein delivery kinetics could be controlled.
**Figure 5****:** IFN-α integrity during *in vitro* release. Symbols indicate the total amount of delivered protein from extrudates comprising 10 % (open symbols) and 20 % (closed symbols) PEG. The bars illustrate the monomer content of delivered IFN-α (brighter bars: extrudates loaded with 10 % PEG, darker bars: extrudates loaded with 20 % PEG). Extrudates with a diameter of 0.5 mm (A), 1.0 mm (B) and 1.9 mm (C) were investigated (average +/-SD; n = 3).
**Figure 6****:** SDS-PAGE of lysozyme extracted from lipidic extrudates. After extrusion the protein was extracted and undergone SDS-PAGE with subsequent Coomassie Blue staining. Lane A and B: lysozyme extracted from lipidic implants, lane C: lysozyme standard, lane D: molecular weight standard. The extrudates formulations were 70% H12/Dynasan 118, 20% PEG, 2.5% lysozyme and 7.5% HP-β-CD (lane A and lane B extrudates received at the beginning and at the end of the extrusion procedure, respectively).
**Figure 7****:** Effect of implant diameter on lysozyme release. The diameter of the extrudates was 0.5 mm (●), 1.0 mm (▲), 1.4 mm (■), or 1.9 mm (◆), respectively (average +/- SD; n = 3).
**Figure 8****:** Lysozyme integrity during *in vitro* release. Symbols indicate the total amount of delivered lysozyme from extrudates with different diameters. The bars illustrate the monomer content of delivered lysozyme (average +/- SD; n = 3).
**Figure 9****:** *In vitro* release kinetics of IFN-α from extrudates prepared by RAM extrusion.
**Figure 10****:** Influence of the implant manufacturing method on the *in vitro* release of IFN-α. *In vitro* release kinetics of IFN-α from solid implants comprising 10 % IFN α-2a/HP-β-CD lyophilisate, 20 % PEG 6000, and 70 % of a lipid powder blend of H12 and tristearin (14 % H12 and 56 % tristearin) prepared by compression (□), by ram extrusion (◊) or by twin screw extrusion (extrudates with a diameter 1.9 mm (●), 1.0 mm (◆), or 0.5 mm (A)). All matrices were loaded with 10 % IFN-a co-lyophilised with HP-β-CD and with 10 % PEG (average +/- SD; n = 3).
**Figure 11****:** Optical appearance of the different solid implants. The lipidic powder blend of 20 % H12 and 80 % tristearin was admixed with 1 % methylene blue in mortar and (A) compressed at 19.8 kN for 30 seconds (B) extruded with a ram extruder or (C) extruded with a twin-screw extruder.
**Figure 12****:** Mechanical strenght of solid implants prepared by various manufacturing methods. Solid implants based on a lipidic powder blend of 20 % H12 and 80 % tristearin (average +/- SD; n = 5) were prepared by compression, ram extrusion or twin screw extrusion. The mechanical strength was determined by Texture Analysis.
**Figure 13****:** Lysozyme release from extruded lipid implants. Incubation temperature was 20°C PBS buffer pH 7.4, 40 rpm (average +/- SD; n = 3).
**Figure 14****:** Lysozyme release from twin screw extruded lipid implants. Incubation temperature was 37°C. PBS buffer pH 7.4, 40 rpm (average +/- SD; n = 3).
**Figure 15****:** Effects of the PEG concentration on the apparent solubility of IFN-α at 37 °C in phosphate buffer pH 7.4 (average +/- SD; n = 3).
**Figure 16****:** A) Effect on NaCl concentration on the apparent solubility of lysozyme at 37 °C in phosphate buffer pH 7.4 (average +/- SD; n = 3). B) Effect of different carboxymethylcellulose (CMC) qualities on the apparent solubility of lysozyme at 37 °C in phosphate buffer pH 7.4 (average +/- SD; n = 3). The molecular weight and the degree of substitution of the used CMC qualities are indicated in the legend.
**Figure 17****:** Effects of the PEG concentration on the apparent solubility of the model IgGl at 37 °C in phosphate buffer pH 7.4 (average +/- SD; n = 3).
**Figure 18****:** Macroscopic appearance of extrudates comprising 2% carboxymethylcellulose (MW 700,000; D.S. 0.9) immediately after the addition of buffer media (A) and after 1 day (B) or 2 days (C) of incubation in PBS buffer at 37°C and 40 rpm.
**Figure 19****:** Lysozyme release from extruded lipid implants containing various amounts of carboxymethylcellulose CMC (MW 700,000, D.S. 0.9), respectively (average +/- SD; n = 3).

### Detailed description of the invention

The present invention relates to an extruded rod-shaped device for sustained delivery of biological substances, obtainable by a process comprising:
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the preparation of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

The term "extruded rod-shaped device", as used herein, refers to a device that is used as a matrix for the delivery of a biological substance. The extruded rod-shaped device preferably has an injectable size, but, if desired, may e.g. be inserted at an administration site by surgical operation. Preferably, the device according to the invention has a diameter size of at least 0.1 mm and/or a length of at least 5 mm. More preferably, the device according to the invention has a diameter size of between 0.2 mm and 2 mm, and most preferably of between 0.4 mm and 1.4 mm. Preferably, the device according to the invention has a length to diameter ratio of at least more than 1, more preferably more than 1.5, and even more preferably more than 2. Preferably, such a device has a good mechanical stability and a breaking strength of at least 10 N, more preferably of at least 15 N, even more preferably of at least 20 N and most preferably of at least 25 N (for a diameter of 1.9 mm). However, the devices according to the present invention shall in no way be limited to a certain, probably convenient size.

With respect to the objects of the present invention, the biological substance-containing device for sustained delivery according to the present invention is in a rod form, or the like. A "rod" in the context of the invention is a 3-dimensional, solid (filled) cylinder with a defined length and a defined diameter. In this context, the term "cylinder" has a number of related meanings. In its most general usage, the word "cylinder" refers to a solid bounded by a closed generalized cylinder and two planes. For example, a cylinder of this sort having two polygonal planes is a prism. It is however to be understood that the planes of such a cylinder are not restricted with regard to shape or form. Generally, the term "diameter" of a subset of a metric space means the least upper bound of the distances between pairs of points in the subset. For instance, for a convex shape in the plane, the diameter is defined to be the largest distance that can be formed between two opposite parallel lines tangent to its boundary.

In general, "rod-shaped" in the context of the invention means that the length is higher than the diameter, preferably at least two times higher, more preferably at least four times higher, even more preferably at least six times higher and most preferably at least eight times higher.

To achieve good extrudability, very homogeneous biological substance distribution, excellent mechanical stability (e.g. an excellent breaking strength), sustained release kinetics with a low burst and mainly stable modification of lipids, the following lipoid composition has to be applied. The composition is composed of at least two lipids, one of them being a high melting, solid lipid or lipoid component and the other a low melting lipid or lipoid component. The lipoid composition may thus comprise low melting lipids in the form of solid, semisolid or liquid lipids at room temperature. The lipoid composition is preferably selected in a way that the overall mixture is solid at room temperature.

Lipids have been referred to as "chemically heterogeneous group of substances, having in common the property of insolubility in water, but solubility in non-polar solvents". More precisely the principal categories of lipids are: fatty acids, fatty acid salts, phospholipids, glycerides, waxes, glycolipids and sterols [Swarbick, J. and Boylan J. C., Lipids in pharmaceutical dosage forms. In Enzyclopedia of pharmaceutical technology, Marcel Dekker, Inc., New york, 1992, pp. 395-441.].

Hence, the "lipid" or "lipoid component", used in the devices according to the present invention, is a water-insoluble substance that is absorbed by the body, does not have side effects and is preferably solid at room temperature. Examples of lipids include fatty acids, monoglycerides, diglycerides, triglycerides, sorbitan fatty acid esters, phospholipids, sphingolipids, cholesterol, waxes, and salts and derivatives thereof.

Available fatty acids include e.g. lauric acid, myristic acid, palmitic acid and stearic acid. Available monoglycerides include e.g. glyceryl laurate, glyceryl myristrate, glyceryl palmitate and glyceryl stearate. Available sorbitan fatty acid esters include e.g. sorbitan myristrate, sorbitan palmitate and sorbitan stearate. Available triglycerides include e.g. trilaurin, trimyristin, tripalmitin, tristearin and triarachin. Available phospholipids include e.g. phosphatidylcholine, phosphatidylethanolamine, phosphatidic acid, phosphatidylserine, phosphatidylglycerol, phosphatidylinositol and cardiolipin. Available sphingolipids include e.g. sphingosine, ceramide and sphinganine.

Most preferably, both the high melting lipid or lipoid component and the low melting lipid or lipoid component are selected from the class of fatty acid mono-, di- and/or triglycerides, and salts and derivatives thereof. In the context of the present invention, the "high melting lipid or lipoid component" has a melting point which is higher than the melting point of the "low melting lipid or lipoid component". The term "melting point" may also refer to the average of a "melting area" of a lipid or lipoid component.

Preferably, the melting point (hereafter called m.p.) of the high melting lipid or lipoid component is above 50°C, more preferably above 60°C, even more preferably above 65°C and most preferably above 70°C. Examples for particularly preferred high melting lipids or lipoid components according to the invention are:
- Dynasan D116 (glyceryl tripalmitate, m.p. 61°C, Sasol GmbH, Witten, Germany)
- Dynasan D118 (gylceryl tristearate, m.p. 71°C, Sasol GmbH, Witten, Germany)
- Dynasan D120 (glyceryl triarachate, m.p. 67°C, Sasol GmbH, Witten, Germany)

Also preferably, the melting point of the low melting lipid or lipoid component is below 50°C, more preferably below 45°C, even more preferably below 40°C and most preferably below 37°C. However, it is also preferred that the melting point of the low melting lipid or lipoid component is above 20°C. Examples for particularly preferred low melting lipids or lipoid components according to the invention are:
- H12 (triglyceride based on 71 % lauric, 27 % myristic and 2 % palmitic acid, m.p. 36°C, Sasol GmbH, Witten, Germany)
- Dynasan D112 (glyceryl trilaureate, m.p. 43°C, Sasol GmbH, Witten, Germany)
- E85 (triglyceride based on 27 % lauric, 71 % myristic and 2 % palmitic acid, m.p. 41 °C, Sasol GmbH, Witten, Germany)
- Miglyol 812 (triglycerides of caprylic (C₈) and capric (C₁₀) fatty acids, m.p. < 25°C, Sasol GmbH, Witten, Germany)

The amount of low melting lipid to be admixed to the high melting lipid can be selected in a wide range allowing fine adjustment of properties like release of the biological substances, mechanical stability, manufacturability etc.

In a preferred embodiment of the invention, the lipids or lipoid components are selected in a way that the weight ratio between the high melting lipid or lipoid component and the low melting lipid or lipoid component in the lipoid composition is between 10:1 1 to 1:10, more preferably between 10:1 1 to 1:1, even more preferably between 8:1 to 2:1, and most preferably between 5:1 to 2:1. In this context, the term "weight ratio" refers to the ratio of mass in a mixture and relates to concentration. Examples for lipoid compositions are shown in Tables 1 and 2.

**Table 1:**

| **Dynasan D118** | **H12** | **Weight ratio** |
|---|---|---|
| 60% (w/w) | 40% (w/w) | 1,5:1 |
| 70% (w/w) | 30% (w/w) | 2,3:1 |
| 80% (w/w) | 20% (w/w) | 4:1 |
| 90% (w/w) | 10% (w/w) | 9:1 |

**Table 2:**

| **Dynasan D118** | **Miglyol 812** | **Weight ratio** |
|---|---|---|
| 75% (w/w) | 25% (w/w) | 3:1 |
| 80% (w/w) | 20% (w/w) | 4:1 |
| 83% (w/w) | 17% (w/w) | 4,9:1 |

In another preferred embodiment of the invention, the lipids or lipoid components are selected in a way that the melting point of the high melting lipid or lipoid component is at least 20°C, preferably at least 25°C, more preferably at least 30°C, and most preferably at least 35°C higher than the melting point of the low melting lipid or lipoid component. Examples for such lipoid compositions are shown in Table 3.

**Table 3:**

| **Low melting lipid** | **High melting lipid** | **Δ m.p. (°C)** |
|---|---|---|
| H12 | Dynasan D116 | 26 |
| Dynasan D112 | Dynasan D118 | 28 |
| E85 | Dynasan D118 | 30 |
| H12 | Dynasan D120 | 31 |
| H12 | Dynasan D118 | 35 |
| Miglyol 812 | Dynasan D118 | > 45°C |

In a preferred embodiment, the device according to the invention comprises a high melting lipid or lipoid component and a low melting lipid or lipoid component, both having a stable lipid modification after extrusion, the stable lipid modification more preferably being a beta modification.

The term "lipid modification" according to the invention refers to different crystallographic types of lipids. Almost all fats and fatty acids possess the ability to form different polymorphs. Dependent on the unit cell structures mono-acid saturated triglycerides are classified into three main crystallographic types. The least stable α-modification is characterised by a loose packing of the hydrocarbon chains in a hexagonal unit cell structure. The intermediate β'-modification reveals an orthorhombic unit cell structure and the most dense packing is achieved with the stable β-form by a triclinic packing [Garti, N., Sato, K., and Editors., Surfactant Science Series, Vol. 31: Crystallization and Polymorphism of Fats and Fatty Acids, 450 Marcel Dekker, New York (1988).].

A polymorphic transformation might occur during the preparation of devices according to the invention. Especially, when the manufacturing process comprises a melting or dissolution step, polymorphism needs to be considered since the crystallisation of lipids follows the so-called Ostwald step rule. Accordingly, the least stable α-form nucleates first, followed by a transition to the intermediate β'-modification, and, finally, the optimal packing is accomplished by a rearrangement to the β-form [Sato, K., Crystallization behaviour of fats and lipids - a review, Chemical Engineering Science. 56: 2255-2265 (2001).].

Surprisingly, the selection of the lipids or lipoid components with different meting points allows extrusion of matrices that are crystalline after processing and possess a neglible amount of unfavourable, instable alpha modification. This leads to storage stable matrices without the risk that re-crystallisation occurs, which would change the physical properties of the extruded device over time. Processes which comprise only one lipid or lipoid component that would be melted during extrusion or the selection of a lipid mixture that would completely be melted under extrusion conditions would both not be leading to a device according to the invention and to a product with the desired features.

The preparation according to the invention comprises at least 50% per weight of a lipoid composition and at least one biological substance. The term "biological substance" as used herein denotes all macromolecular biological substances. The term "macromolecular biological substance" in the context of the invention thus denotes a biological substance which has a molecular weight of preferably more than 1,000 daltons, more preferably more than 2,000 daltons. Such biological substances include vaccines, serums, biological drugs, adjuvants to enhance or modulate a resulting immune response, vitamin antagonists, medications, and all substances derived from and/or related to the foregoing substances. Preferably, the term "biological substances" denotes a macromolecular biological substance which comprises a protein, polypeptide, peptide or nucleic acid molecule, or a salt or derivative thereof.

The term "derivative" of a compound as used herein means a chemically modified compound wherein the chemical modification takes place at one or more functional groups of the compound. The derivative however is expected to retain the pharmacological activity of the compound from which it is derived.

Furthermore, wherever the generic term "biological substance" is used herein it is also intended to mean species which employ any or more of the individual biological substances as defined and/or alluded to herein. Hence, the term "biological substances" also includes *inter alia* virus-like particles and viruses, microorganisms and their spores, and any other kind of multimers.

However, the term "biological substance" does not refer to a low molecular weight chemical compound. The term "low molecular weight chemical compound", as used herein, denotes a molecule, preferably an organic molecule, comprising at least two carbon atoms, but preferably not more than seven carbon bonds, and preferably having a molecular weight in the range between 100 and 2,000 daltons, more preferably between 100 and 1,000 daltons, and optionally including one or two metal atoms. Examples of such molecules include *inter alia* imidazoles, indoles, isoxazoles, oxazoles, pyridines, pyrimidines, and thiazoles.

The term "biological substance" according the present invention also comprises biological substances as described above which have been incorporated into nanoparticles or microparticles. A "nanoparticle" (or nanopowder or nanocluster or nanocrystal) is a small particle with at least one dimension less than 100 nm. At the small end of the size range, nanoparticles are often referred to as clusters. Nanospheres, nanorods, and nanocups are just a few of the shapes that have been grown. Such nanoscale particles are used in biomedical applications as drug carriers or imaging agents and are generally known in the art. A prototype nanoparticle of semi-solid nature includes e.g. the liposome. Various types of liposome nanoparticles are currently used clinically as delivery systems for anticancer drugs and vaccines. "Microparticles" are such particles between 0.1 and 100 µm in size.

As used herein, the term "amino acid" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The terms "polypeptide", "peptide", "oligopeptide", "polypeptide", "gene product", "expression product" and "protein" are used interchangeably herein to refer to a polymer or oligomer of consecutive amino acid residues.

The term "nucleic acid" refers to deoxyribonucleotides or ribonucleotides and polymers or hybrids thereof in either single-or double-stranded, sense or antisense form. Unless otherwise indicated, a particular nucleic acid sequence also implicitly encompasses conservatively modified variants thereof (e. g., degenerate codon substitutions) and complementary sequences, as well as the sequence explicitly indicated. The term "nucleic acid" is used inter-changeably herein with "gene", "cDNA, "mRNA", "oligonucleotide," and "polynucleotide".

With respect to the objects of the present invention, a "protein" capable of being contained in the extruded rod-shaped device of the present invention includes a biologically active protein or peptide, or derivatives and mutants thereof, and may be naturally occurring, recombinantly manipulated or synthesized. Also, the protein may possess a variety of modifications, such as an addition, substitution, or deletion of an amino acid or domain, or glycosylation, and is not specifically limited. Hence, as used herein, the term "proteins" also comprises lipoproteins and glycoproteins. The term "proteins" shall also include e.g. protein aggregates.

Examples of "proteins" include human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, toxins, etc., and virus-derived vaccine antigens. Preferred are human serum albumin, human growth hormone, interferon alpha, erythropoietin, colony stimulating factors, etc.

Preferably, the extruded device according to the invention also comprises at least one excipient which modifies the release of the at least one biologically active substance from the implantable device and/or modifies the biodegradation of the lipids or lipoid components of the implantable device and/or stabilises the biological substance during manufacturing, storage and release. Furthermore, the excipient might modify the solubility of the biological substance and/or can itself feature slow dissolution behavior, i.e. the excipient can e.g. itself be sustained dissolved. The excipient can e.g. be a hydrophilic polymer, a sugar, a polyol, a surfactant and/or a water-soluble salt or any other excipient known to achieve the above mentioned purposes.

The "hydrophilic polymer" used in the device of the present invention is a polymeric substance that is admixed with the lipoid components. The hydrophilic polymer is stably present between hydrophobic lipid molecules. This hydrophilic polymer may *inter alia* protect and stabilize biological substances, prevent denaturation of proteins and/or nucleic acids, and induce the stable release of biological substances. The biological substances are released when the lipid is exposed to body fluids, dissolved, and degraded or absorbed. In this context, the excipient modifies the release of the at least one biologically active substance from the extruded rod-shaped device by leaching out of the implantable device, thereby facilitating the formation of pores. The hydrophilic polymer can prevent a protein and/or a nucleic acid from being denatured during the preparation of the device as well as during release after the device is administered to or into the body. Also, the hydrophilic polymer can protect a biological substance against degradation and aggregation as well as against denaturation, can enhance the *in vivo* activity of the biological substance, and/or can maintain the sustained release of the biological substance.

In a preferred aspect, the present invention employs, as the hydrophilic polymer, a substance preferably having a molecular weight of more than about 1,000 daltons, more preferably of more than 2,000 daltons, which is selected from the group consisting of polyethylene glycol, polyvinylpyrrolidone, polyvinylalcohol, dextran, dextran sulfate, chondroitin sulfate, dermatan sulfate, heparan sulfate, keratan sulfate, hyaluronic acid, chitosan, albumin, fibrin, cyclodextrin and mixtures thereof. Most preferred are polyethylene glycol or cyclodextrin.

In some embodiments of the invention, the excipient modifying the release of the at least one biologically active substance from the implantable device and/or modifying the biodegradation of the lipids or lipoid components of the implantable device can be selected from the group consisting of carboxymethylcellulose, gelantine and starch.

In the context of the invention, polyethylene glycol (PEG) and polyethylene oxide (PEO) are polymers composed of repeating subunits of identical structure, called monomers. Poly (ethylene glycol) or poly (ethylene oxide) refers to an oligomer or polymer of ethylene oxide. The two names are chemically synonymous, but historically PEG has tended to refer to shorter polymers, PEO to longer. PEG and PEO are liquids or low-melting solids, depending on their molecular weights. Both are prepared by polymerization of ethylene oxide. While PEG and PEO with different molecular weights find use in different applications and have different physical properties (e.g. viscosity) due to chain length effects, their chemical properties are nearly identical. Derivatives of PEG and PEO are in common use, the most common derivative being the methyl ether (methoxypoly (ethylene glycol)), abbreviated mPEG. The numbers that are often included in the names of PEGs and PEOs indicate their average molecular weights, e.g. a PEG with n=80 would have an average molecular weight of approximately 3500 daltons and would be labeled PEG 3500. Most PEGs and PEOs include molecules with a distribution of molecular weights, i.e. they are polydisperse. The size distribution can be characterized statistically by its weight average molecular weight (Mw) and its number average molecular weight (Mn), the ratio of which is called the polydispersity index (Mw/Mn). PEG is soluble in water, methanol, benzene, dichloromethane and is insoluble in diethyl ether and hexane.

Cyclodextrins (sometimes called cycloamyloses) make up a family of cyclic oligosaccharides, composed of 5 or more α-D-glucopyranoside units linked 1->4, as in amylose (a fragment of starch). Typical cyclodextrins contain a number of glucose monomers ranging from six to eight units in a ring, creating a cone shape, thus denoting: α-cyclodextrin (six sugar ring molecule), β-cyclodextrin (seven sugar ring molecule) and γ-cyclodextrin (eight sugar ring molecule). Cyclodextrins are produced from starch by means of enzymatic conversion. Typical cyclodextrins are constituted by 6-8 glucopyranoside units and can be topologically represented as toroids with the larger and the smaller openings of the toroid exposing to the solvent secondary and primary hydroxyl groups, respectively. Because of this arrangement, the interior of the toroids is not hydrophobic, but considerably less hydrophilic than the aqueous environment and thus able to host other hydrophobic molecules. On the contrary the exterior is sufficiently hydrophilic to impart cyclodextrins (or their complexes) water solubility.

A certain number of additives may be added to the polymers in order to optimise their chemical, physical and mechanical properties in order to adapt them for the intended use. These additives include *inter alia* sodium benzoate, fatty acid esters or salts, trisodium phosphate, liquid paraffin, zinc oxide, calcium stearate, zinc stearate.

Alternatively or additionally, the excipient may have a lipase activity, thereby modifying the biodegradation of the lipids or lipoid components comprised by the implantable device. A lipase is a water-soluble enzyme that catalyzes the hydrolysis of ester bonds in water-insoluble, lipid substrates. Lipases thus comprise a subclass of the esterases. The term "lipase" includes inter alia pancreatic lipase, lysosomal lipase, hepatic lipase, lipoprotein lipase, hormone-sensitive lipase, gastric lipase, endothelial lipase, pancreatic lipase related protein 2, pancreatic lipase related protein 1 and lingual lipase. The term "lipase" also comprises phospholipases, i.e. an enzyme that converts phospholipids into fatty acids and other lipophilic substances.

Alternatively or additionally, a lipid excipient can be added that facilitates the biodegradation of the lipids or lipoid components comprised by the implantable device (i) by the formation of a mixed-lipid phase, (ii) by the formation of an eutectic phase, (iii) by its amphiphilic character, or by (iv) its high susceptibility to lipase cleavage. Such an excipient may be selected from the group consisting of mixed and mono-acid triglycerides, diglycerides, monoglycerides, fatty acids, and phospholipids.

A "salt" in the context of the invention is generally defined as the product formed from the neutralisation reaction of acids and bases. Salts are ionic compounds composed of cations (positively charged ions) and anions (negative ions) so that the product is electrically neutral (without a net charge). These component ions can be inorganic such as chloride, as well as organic such as acetate and monoatomic ions such as fluoride, as well as polyatomic ions such as sulfate. There are several varieties of salts. Salts that produce hydroxide ions when dissolved in water are basic salts and salts that produce hydronium ions in water acid salts. Neutral salts are those that are neither acid nor basic salts. Zwitterions contain an anionic center and a cationic center in the same molecule but are not considered to be salts. Common salt-forming cations include ammonium, calcium, iron, magnesium, potassium, pyridinium, quaternary ammonium and sodium. Common salt-forming anions (and the name of the parent acids in parentheses) include: acetate (acetic acid), carbonate (carbonic acid), chloride (hydrochloric acid), citrate (citric acid), hydroxide (water), nitrate (nitric acid), oxide (water), phosphate (phosphoric acid), succinate (succinic acid), maleate (maleinic acid), trishydroxymethylaminomethane (tris) and sulfate (sulfuric acid).

The term "sugar" as used herein means a carbohydrate. Carbohydrates include compounds such as monosaccharides, oligosaccharides, polysaccharides, glycoproteins, glycolipids and the like. Carbohydrates of the present invention also include carbohydrate-nucleoside hybrid molecules, such as carbohydrate-oligonucleotide hybrid molecules. As used herein, the term "monosaccharide" includes a compound that is the basic unit of a carbohydrate, consisting of a single sugar. Monosaccharides include glucose, glyceraldehydes, ribose, mannose, galactose and the like. As used herein, the term "oligosaccharide" refers without limitation to several (e.g., two to ten) covalently linked monosaccharide units. Oligosaccharides include disaccharides (i.e., two monosaccharide units) such as sucrose, lactose, maltose, isomaltose, cellobiose and the like. Oligosaccharides are often associated with proteins (i.e., glycoproteins) and lipids (i.e., glycolipids).

Oligosaccharides form two types of attachments to proteins: N-glycosidic and O-glycosidic. As used herein, the term "polysaccharide" refers without limitation to many (e.g., eleven or more) covalently linked monosaccharide units. Polysaccharides can have molecular masses ranging well into millions of daltons. Polysaccharides include cellulose, chitin, starch, glycogen, glycosaminoglycans (e.g., hyaluronic acid, chondroitin-4-sulfate, chondroitin-6-sulfate, dermatan sulfate, keratin sulfate, heparin and the like) and the like.

As used herein, the term "polyol" is intended to include any linear, cyclic, or aromatic compound containing at least four free esterifiable hydroxyl groups. For example, suitable polyols can be selected from the following classes: saturated and unsaturated straight and branched chain linear aliphatics; saturated and unsaturated cyclic aliphatics, including heterocyclic aliphatic; or mononuclear or polynuclear aromatics, including heterocyclic aromatics. Carbohydrates and nontoxic glycols are preferred polyols. Monosaccharides suitable for use herein include, for example, mannose, galactose, arabinose, xylose, ribose, apiose, rhamnose, psicose, fructose, sorbose, tagitose, ribulose, xylulose, and erythrulose. Oligosaccharides suitable for use herein include, for example, maltose, kojibiose, nigerose, cellobiose, lactose, melibiose, gentiobiose, turanose, rutinose, trehalose, sucrose and raffinose. Polysaccharides suitable for use herein include, for example, amylose, glycogen, cellulose, chitin, insulin, agarose, zylans, mannan and galactans. Although sugar alcohols are not carbohydrates in a strict sense, the naturally occurring sugar alcohols are so closely related to the carbohydrates that they are also preferred for use herein. The sugar alcohols most widely distributed in nature and suitable for use herein are sorbitol, mannitol and galactitol.

Preferred carbohydrates and sugar alcohols include trehalose, saccharose and mannitol.

"Surfactants", also known as tensides, are wetting agents that lower the surface tension of a liquid, allowing easier spreading, and lower the interfacial tension between two liquids. Surfactants are usually organic compounds that are amphipathic, meaning they contain both hydrophobic groups (their "tails") and hydrophilic groups (their "heads"). Therefore, they are soluble in both organic solvents and water. A surfactant can be classified by the presence of formally charged groups in its head. A nonionic surfactant has no charge groups in its head. The head of an ionic surfactant carries a net charge. If the charge is negative, the surfactant is more specifically called anionic; if the charge is positive, it is called cationic. If a surfactant contains a head with two oppositely charged groups, it is termed zwitterionic.

Ionic surfactants include anionic surfactants (based on sulfate, sulfonate or carboxylate anions), such as sodium dodecyl sulfate (SDS), ammonium lauryl sulfate, and other alkyl sulfate salts, sodium laureth sulfate (also known as sodium lauryl ether sulfate (SLES)), alkyl benzene sulfonate, soaps and fatty acid salts; cationic surfactants (based on quaternary ammonium cations), such as cetyl trimethylammonium bromide (CTAB) and other alkyltrimethylammonium salts, cetylpyridinium chloride (CPC), polyethoxylated tallow amine (POEA), benzalkonium chloride (BAC), benzethonium chloride (BZT); and zwitterionic (amphoteric) surfactants, such as dodecyl betaine, dodecyl dimethylamine oxide, cocamidopropyl betaine, coco ampho glycinate. Nonionic surfactants include alkyl poly(ethylene oxide), copolymers of poly(ethylene oxide) and poly(propylene oxide) (commercially called Poloxamers or Poloxamines), alkyl polyglucosides, fatty alcohols, cocamide MEA, cocamide DEA and cocamide TEA.

Preferred surfactants include polysorbates (such as Tween) and Poloxamers (such as Pluronics).

Mechanical stability (e.g. breaking strength) of the device is surprisingly achieved by the process of the invention. The present invention thus further relates to a method of producing a rod-shaped device for sustained delivery of a biological substance, comprising
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the composition of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

The preparation according to the invention comprises the lipoid composition and the at least one biological substance and, in a preferred embodiment, further comprises at least one excipient. It is possible to provide the excipients and the biological substances in a mixture and add the pre-mixture to the lipoid composition or to provide the components separately and mix it all together. The excipients as defined above can provide different stabilising, release controlling, redissolution and other properties to the device according to the invention.

The preparation is then formed into an extrudate by extrusion at a temperature, wherein the low meting lipid or lipoid component is at least softened in a way that the extruder can mix the entire preparation homogeneously, and extrusion through a nozzle occurs. The presence of non-molten lipid compounds during the process is typical for the process according to the invention. Surprisingly, rod-shaped devices obtained from such extrudates have excellent mechanical properties and low initial release burst rates and sustained drug release over long periods of time. Also surprisingly, such extruded rod-shaped devices have even longer release and less initial burst rates compared to devices produced from such mixtures by compression into geometrically larger disk-shaped implants (tablets).

The extrusion process may be carried out at a temperature below the melting point of the higher melting lipid, keeping much temperature stress off the biological substances. Surprisingly, due to the high mixing efficiency of the extrusion process, a complete pre-mixture of the excipients, the biological substances and the lipids by processes like a rather complicated co-lyophilisation process etc. can be avoided. In a preferred embodiment of the invention, extrusion is carried out at a temperature which is at or 1°C to 25°C above the melting point of the low melting lipid or lipoid component, preferably between 1 °C and 20°C above the melting point of the low melting lipid or lipoid component, and more preferably between 1°C and 10°C above the melting point of the low melting lipid or lipoid component. Particularly preferably, extrusion is carried out at a temperature which is between 40°C and 60°C.

"Extrusion" is a manufacturing process used to create long objects of a fixed cross-sectional profile, called extrudates. A material, often in the form of a billet, is pushed and/or drawn through a die of the desired profile shape. For that purpose at least two main components are necessary: (1) a transport system that may impart a mixing function and (2) a die system, which forms the material. The pressure required for extrusion depends on the design of the die, on the extrusion rate, and in particular on the rheological characteristics of the preparation. Extrusion may be continuous (producing indefinitely long material) or semi-continuous (producing many short pieces). With respect to the method used to adapt the viscosity, extrusion can be classified into molten systems (hot-melt extrusion) and semisolid systems. Semisolid systems are generated by dispersing a high portion of solid material in a liquid phase [Swarbick, J. and Boylan J.C. Extrusion and Extruder. In Enzyclopedia of pharmaceutical technology, Marcel Dekker, Inc., New york, 1992, pp. 395-441.]. This technique is widely used to prepare granules or pellets, whereas for the preparation of parenteral controlled release devices preferably the hot melt extrusion technique is applied [Kissel, T., Li, Y., and Unger, F., ABA-triblock copolymers from biodegradable polyester A-blocks and hydrophilic poly(ethylene oxide) B-blocks as a candidate for in situ forming hydrogel delivery systems for proteins, Advanced Drug Delivery Reviews 54: 99-134 (2002).].

Various extruder types can be distinguished. A "ram extruder" consists of a barrel that is pre-filled with the powder mixture. By means of a piston (or ram) the material is forced through the die at the bottom of the barrel. Compared to screw extrusion, ram extrusion is a non-continuous procedure. Since only small amounts of substance are necessary, ram extrusion is mostly used in laboratory scale.

The "screw type extruder" consists of at least one rotating screw inside a stationary cylindrical barrel. At the end of the barrel a die is connected to shape the device. The extrusion channel can be divided into three distinct sections. Within the first zone, the feed zone, the extruder is loaded. After the feeding zone the transition zone follows. Within this zone the pressure increases due to the reduction of the thread pitch while maintaining a constant flight depth or due to the reduction flight depth while maintaining the thread pitch. Thus, a compression of the material takes place. Finally, the material arrives at the metering zone as a homogeneous plastic melt suitable for extrusion. In this last section the pulsating flow is reduced and a uniform delivery rate through the die is achieved. Typical screw type extruders are single- or twin-screw extruders. In a preferred embodiment of the invention, the extruder is equipped with at least one pair of fully intermeshing, co-rotating, extruder elements. Such extruders include *inter alia* twin screw extruders.

The extruded device may then be obtained from the extrudate by cutting or breaking-off the extrudate into small sections. For that purpose any apparatus which is capable of cutting or breaking-off the extrudate into sections of substantially identical size and shape can be used. For example, the industry has experienced the use of cutting knives which slice the extrudate from the extruder die plate as it emerges. An alternative method of cutting extrudates comprises extending material into a rapidly spinning disc. The rotary motion of the disc, when it is hit by the extrudate, causes breakage of the extrudate at the point where it emerges from the die.

The present invention further relates to an extruded rod-shaped device according to the invention, or which is produced by a method according to the invention, for use as a parenteral delivery system with sustained delivery of biological substances. The present invention also relates to the use of the extruded rod-shaped device according to the invention, or which is produced by a method according to the invention, as a parenteral delivery system for sustained delivery of biological substances. Preferably, such parenteral delivery systems are for subcutaneous, nasal, pulmonary, rectal, vaginal, dermal, buccal administration. The extruded rod-shaped device preferably has an injectable size and may be inserted by injection, but, if desired, may be inserted at an administration site by surgical operation or by other means. Particularly preferred is the subcutaneous administration, wherein the extruded device is inserted into a syringe needle, and subcutaneously inserted using a syringe having a piston fitted into the needle.

Hence, the term "administration" generally refers to a method of placing or introducing the delivery system to or into a desired site. The placing of an extruded device can be by any pharmaceutically accepted means such as placing it within the buccal cavity, inserting, implanting, attaching, etc. These and other methods of administration are known in the art. Thus, the term "administration" shall include injection, insertion, placement, attachment, surgery, needle-free injection and any other pharmaceutically accepted means.

It is preferred that the at least one biological substance is delivered over a period of at least one week. More preferably the at least one biological substance is delivered over a period of at least two weeks, and even more preferably over a period of at least three weeks. In a specific embodiment of the invention implants can provide release rates for up and above 60 days. In the context of the invention, the term "delivery" shall mean the *in vitro* and/or *in vivo* release behaviour of the extruded rod-shaped device. The *in vitro* release behaviour may be measured as exemplified below.

The extruded rod-shaped devices according to the invention can be used as a parenteral delivery system with sustained biodistribution for biological substances; as a delivery system for nasal, pulmonary, rectal, vaginal, dermal and/or buccal administration in humans and animals; as a delivery system for subcutanous administration in humans and animals; and/or as a delivery system for use in agricultural applications or other *in vitro* release situations where biological substances shall be sustained released over time.

Alternatively or additionally, the extruded rod-shaped devices of the invention can be used as a delivery system that allows the temperature-dependent modulate release of the biological substance at the site of action (temperature variations can be adjusted by a separate heat treatment or in combination with certain diseases (such as inflammatory reactions) or treatments (such as laser therapy)). For instance, the release of the biological substance could be switched on by increasing the temperature of the implanted rod-shaped device. This could e.g. be achieved by placing or applying a heating element in close vicinity to the implanted inventive device (e.g., heating the skin section of a patient where the device has been implanted). Suitable heating elements are known to the skilled person and may, *inter alia,* include a infrared lamp, a laser or a hot-water bottle.

Furthermore, fever is a frequent medical sign that describes an increase in internal body temperature to levels above normal. Fever is most accurately characterized as a temporary elevation in the body's thermoregulatory set-point, usually by about 1-2 °C. Hence, the extruded rod-shaped devices of the invention may further be used as a delivery system that allows the temperature-dependent modulate release of the biological substance depending on the body temperature, i.e. the device may be manufactured in that the release is switched on or increased if there is a temporary elevation in the body's thermoregulatory set-point and is switch off or decreased if the internal body temperature decreases to normal levels. Hence, the extruded rod-shaped devices according to the invention have the advantage that the release rate can be controlled by modulating the temperature of the device or their surroundings.

Suitable pharmaceutical dosage forms as well as methods for their preparation are well established in the art (see, for example, A.R. (2000) Remington: The Science and Practice of Pharmacy, 20th Ed., Lippincott Williams & Wilkins, Philadelphia, PA; Niazi, S.K. (2004) Handbook of Pharmaceutical Manufacturing Formulations, CRC Press, Boca Raton, FL).

The process according to the invention can be easily up-scaled towards practically unlimited dimensions as extrudes of the kind used are well comparable to large and very large scale machinery.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent, more preferably 5 percent up or down (higher or lower). As used herein, the word "or" means any one member of a particular list and also includes any combination of members of that list.

It is to be understood that this invention is not limited to the particular methodology, protocols, and reagents described as such. It is also to be understood that the terminology used herein is for the purpose of describing particular embodiments only, and is not intended to limit the scope of the present invention, which will be limited only by the appended claims. It must be noted that as used herein and in the appended claims, the singular forms "a," "and," and "the" include plural reference unless the context clearly dictates otherwise. Thus, for example, reference to "a lipid" is a reference to one or more lipids and includes equivalents thereof known to those skilled in the art, and so forth.

The invention is further described by the figures and the following examples, which are solely for the purpose of illustrating specific embodiments of this invention, and are not to be construed as limiting the scope of the invention in any way.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

### Examples

### Example 1

The extruded rod-shaped devices shown in the examples have been manufactured as follows:
**A.)**
   - 16 % H 12 (triglyceride based on 71 % lauric, 27 % myristic and 2 % palmitic acid, m.p. 36°C, Sasol GmbH, Witten, Germany)
   - 64 % Dynasan 118 (gylceryl tristearate, m.p. 71°C, Sasol GmbH, Witten, Germany)
   - 10 % PEG 6000
   - 10 % IFN α-2a lyophilized with HP-ß-CD in a ratio of 1 to 3
**B.)**
   - 14 % H 12 (triglyceride based on 71 % lauric, 27 % myristic and 2 % palmitic acid, m.p. 36°C, Sasol GmbH, Witten, Germany)
   - 56 % Dynasan 118 (gylceryl tristearate, m.p. 71°C, Sasol GmbH, Witten, Germany)
   - 20 % PEG 6000
   - 10 % IFN α-2a lyophilized with HP-ß-CD in a ratio of 1 to 3
   The lipid powder comprising the low and the high melting point lipid was prepared by grinding in a mortar. Subsequently, the obtained blend was admixed with 10 % IFN-α/HP-β-CD lyophilisate and PEG, optionally 10 % or 20 %. Extrusion was performed using a twin screw extruder (MiniLab Micro Rheology Compounder, Thermo Haake GmbH Karlsruhe, Germany). The extruder was heated to 40 °C prior filling. The rotation speed of the screws was fixed at 40 rpm. Then the extruder was manually filled with the lipid blend. Extrusion was performed with closed bypass channel to allow a direct extrusion without circulating. In order to prepare extrudates of different sizes, dies with a diameter of 0.5 mm or 1 mm were fixed in front of the standard extruder outlet (diameter 2.0 mm). The extruded strands were cut into pieces with a length of approximately 2.3 cm.
**C.)**
   - 16 % H 12 (triglyceride based on 71 % lauric, 27 % myristic and 2 % palmitic acid, m.p. 36°C, Sasol GmbH, Witten, Germany)
   - 64 % Dynasan D120 (glyceryl triarachate, m.p. 67°C, Sasol GmbH, Witten, Germany)
   - 10 % PEG 6000
   - 10 % IFN α-2a lyophilized with HP-ß-CD in a ratio of 1 to 3
   Extrusion was carried out as described above.
**D.)**
   - 14 % H 12 (triglyceride based on 71 % lauric, 27 % myristic and 2 % palmitic acid, m.p. 36°C, Sasol GmbH, Witten, Germany)
   - 56 % Dynasan D120 (glyceryl triarachate, m.p. 67°C, Sasol GmbH, Witten, Germany)
   - 20 % PEG 6000
   - 10 % IFN α-2a lyophilized with HP-ß-CD in a ratio of 1 to 3
   Extrusion was carried out as described above.
**E.)**
   - 16 % D112 (glyceryl trilaureate, m.p. 43°C, Sasol GmbH, Witten, Germany)
   - 64 % Dynasan 118 (gylceryl tristearate, m.p. 71°C, Sasol GmbH, Witten, Germany)
   - 10 % PEG 6000
   - 10 % IFN α-2a lyophilized with HP-ß-CD in a ratio of 1 to 3
   Manufacturing of extrudates was performed as described above, but the extruder was heated to 47 °C.
**F.)**
   - 14 % D112 (glyceryl trilaureate, m.p. 43°C, Sasol GmbH, Witten, Germany)
   - 56 % Dynasan 118 (gylceryl tristearate, m.p. 71°C, Sasol GmbH, Witten, Germany)
   - 20 % PEG 6000
   - 10 % IFN α-2a lyophilized with HP-β-CD in a ratio of 1 to 3
   Manufacturing of extrudates was performed as described above, but the extruder was heated to 47 °C.
**G.)**
   - 16 % E85 (triglyceride based on 27 % lauric, 71 % myristic and 2 % palmitic acid, m.p. 41°C, Sasol GmbH, Witten, Germany)
   - 64 % Dynasan 118 (gylceryl tristearate, m.p. 71°C, Sasol GmbH, Witten, Germany)
   - 10 % PEG 6000
   - 10 % IFN α-2a lyophilized with HP-ß-CD in a ratio of 1 to 3
   Manufacturing of extrudates was performed as described above, but the extruder was heated to 45 °C.
**H.)**
   - 14 % E85 (triglyceride based on 27 % lauric, 71 % myristic and 2 % palmitic acid, m.p. 41°C, Sasol GmbH, Witten, Germany)
   - 56 % Dynasan 118 (gylceryl tristearate, m.p. 71°C, Sasol GmbH, Witten, Germany)
   - 20 % PEG 6000
   - 10 % IFN α-2a lyophilized with HP-ß-CD in a ratio of 1 to 3
   Manufacturing of extrudates was performed as described above, but the extruder was heated to 45 °C.
**I.)**
   - 14 % H 12 (triglyceride based on 71 % lauric, 27 % myristic and 2 % palmitic acid, m.p. 36°C, Sasol GmbH, Witten, Germany)
   - 56 % Dynasan D118 (gylceryl tristearate, m.p. 71°C, Sasol GmbH, Witten, Germany)
   - 20 % PEG 6000
   - 2.5 % Lysozyme
   - 7.5 % HP-ß-CD
   Manufacturing of lipidic extrudates as described above for IFN-α, but 2.5 % of lysozyme powder were directly blended with the hydrophilic excipients and the lipids.

### Example 2

Wide-Angle X-Ray Scattering (WAXS) was performed in order to investigate the lipid modification of the produced extruded rod-shaped devices (according to example 1A and 1B). Lipidic controlled release systems, the pure lipids as well as lipidic blend before manufacturing were ground. Wide-angle X-ray scattering (WAXS) was performed by an X-ray Diffractometer XRD 3000 TT (Seifert, Ahrensberg, Germany), equipped with a copper anode (40 kV, 30 mA wavelength 0,154178 nm). Experiments were conducted at 0.05° (2 theta) within a 5° - 40° range.

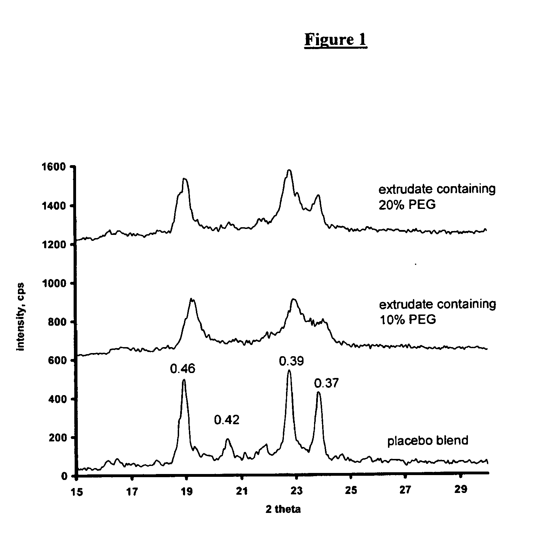

As shown in Figure 1, the freshly prepared extrudates revealed the short-spacings typical for the stable β-modification at 0.46, 0.38 and 0.37 nm [Garti, N., Sato, K., and Editors., Surfactant Science Series, Vol. 31: Crystallization and Polymorphism of Fats and Fatty Acids, 450 Marcel Dekker, New York (1988).]. Therfore, the absence of the α-modification after extrusion was confirmed by WAXS experiments.

### Example 3

The experiment dealt with a critical point of the manufacturing procedure: the effects of extrusion on protein stability. IFN-α loaded extrudates were prepared based on the formulations presented before (Example 1A and 1B).

After extrusion, the protein was extracted with an aqueous extraction method [Mohl, S., The Development of a Sustained and Controlled Release Device for Pharmaceutical Proteins based on Lipid Implants, No (2003).]. Briefly, the protein-loaded matrix was ground in an agate mortar. Subsequently, 50 mg of the sample were suspended in 1 mL pH 7.4 isotonic 0.01 M sodium phosphate buffer containing 0.05 % (wt/vol) sodium azide and 1 % (wt/vol) polysorbate 20 (PBST). After gentle agitation for 2 hours the samples were centrifuged at 5000 rpm for 5 minutes (4K15 laboratory centrifuge; Sigma, Osterode, Germany). The samples were analysed by SDS-PAGE with subsequent silver staining.

SDS-PAGE was conducted under non-reducing conditions using an XCell II Mini cell system (Novex, San Diego, CA). The protein solutions were diluted in a pH 6.8 tris- buffer, containing 2 % SDS and 2 % glycerine. Samples were denatured at 90°C for 30 min and subsequently 20 µL were loaded into the gel wells (NUPAGE Novex 10 % Bis Pre-Cast Gel 1.0 mm; Invitrogen, Groningen, The Netherlands). Electrophoresis was performed in a constant current mode of 30 mA in a tris-glycine/SDS running buffer (MES running buffer; Invitrogen, Groningen, The Netherlands). Gel staining and drying was accomplished with a silver staining kit (SilverXpress) and a drying system (DryEase), both provided from Invitrogen, Groningen, Netherlands.

The presence of a minor amount of dimer specimen was evident in all IFN-α samples (Figure 2). The dimer fraction detected in all extruded samples was already present in the IFN-α bulk material and in the lyophilisates used for extrusion. In comparison to the protein standard and the lyophilised raw material the extrusion process induces no further aggregation or fragmentation.

### Example 4

FTIR-spectroscopy has been suggested to analyse the secondary structure of proteins embedded within controlled release devices [Fu, K., Griebenow, K., Hsieh, L., Klibanov, A. M., and Langer, R., FTIR characterization of the secondary structure of proteins encapsulated within PLGA microspheres, Journal of Controlled Release. 58: 357-366 (1999); van de Weert, M., van't Hof, R., van der Weerd, J., Heeren, R. M. A., Posthuma, G., Hennink, W. E., and Crommelin, D. J. A., Lysozyme distribution and conformation in a biodegradable polymer matrix as determined by FTIR techniques, Journal of Controlled Release. 68: 31-40 (2000)]. This method inheres the benefit that extraction of the protein is not necessary as the protein structure is directly studied within the delivery system. As many proteins revealed spectral changes upon lyophilisation [Carpenter, J. F., Prestrelski, S. J., and Dong, A., Application of infrared spectroscopy to development of stable lyophilized protein formulations, European Journal of Pharmaceutics and Biopharmaceutics. 45: 231-238 (1998)] first the KBr transmission spectra of lyophilised IFN-α were recorded.

For transmission measurements 2 mg of lyophilised IFN-α, lipid/protein/PEG blend before or after extrusion were mixed with 150 mg KBr, respectively. After compression (78.4 kN for 2 minutes) the KBr pellet was fixed in the sample holder (tensor 27, Bruker Optik, Ettlingen, Germany) and spectra were collected with a total of 256 scans at a resolution of 2 cm⁻¹. The obtained absorbance spectra were automatically baseline corrected (OPUS, Bruker Optik, Ettlingen, Germany). Background correction was performed manually. The obtained spectra were vector-normalised and analysed by second derivatisation in the amid I band region (OPUS, Bruker Optik, Ettlingen, Germany).

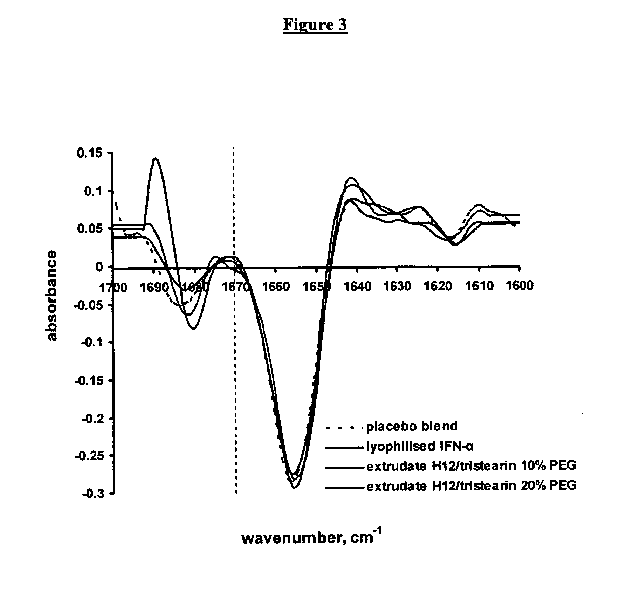

The second derivative transmission spectra of IFN-α lyophilised with HP-β-CD and that of the protein in solution are shown in Figure 3. The band at 1653 cm⁻¹ was largely unaffected by the extrusion procedure. Thus, extrusion of IFN α-2a embedded in a H12/Dynasan 118 blend comprising either 10% or 20% PEG does not induce important changes in the secondary protein structure.

### Example 5

The *in vitro* release behaviour of IFN-α from lipidic extrudates (example 1A and example 1B) was investigated. In order to facilitate the creation of an interconnected pore network enabling complete protein release the extrudates were loaded with 10 % or 20 % PEG, respectively. In addition, the influence of different extrudate diameters on the protein liberation was studied.

The protein-loaded implants were placed into TopPac vials (cycloolefin copolymer vials; Schott GmbH, Mainz, Germany). Depending on the mass of the studied implants the vials were filled with isotonic 0.01 M phosphate buffer pH 7.4 containing 0.05 % (wt/vol) sodium azide (PBS). For extrudates with a diameter of 0.5 or 1 mm, 1.0 mL of buffer was added, for extrudates with a diameter of 1.9 mm the volume of buffer was increased to 2.0 mL. The vials were placed in a horizontal shaker (40 rpm, 37 °C, Certomat^{®}IS; B. Braun Biotech International, Göttingen, Germany). At predetermined time points, the release medium was completely exchanged and the released amount of IFN-α was determined as described below. The frequent buffer exchange as well as the absence of acidic/basic degradation or release products ensured a constant pH in the release media throughout the experiments.

Protein concentration was measured by size-exclusion chromatography (SE-HPLC) using a TSKgel (G3000SWXL, 7.8 mm x 30.0 mm column; Tosoh Biosep, Stuttgart, Germany). The mobile phase consisted of 120 mM disodium hydrogen phosphate dihydrate, 20 mM sodium dihydrogen phosphate and 4 g/L sodium chloride (adjusted to pH 5.0 with hydrochloric acid), the flow rate was 0.5 mL/min, and IFN-α was detected spectrophotometrically (λ = 215 nm, UV 1000; Thermo Electron Cooperation, Dreieich, Germany).

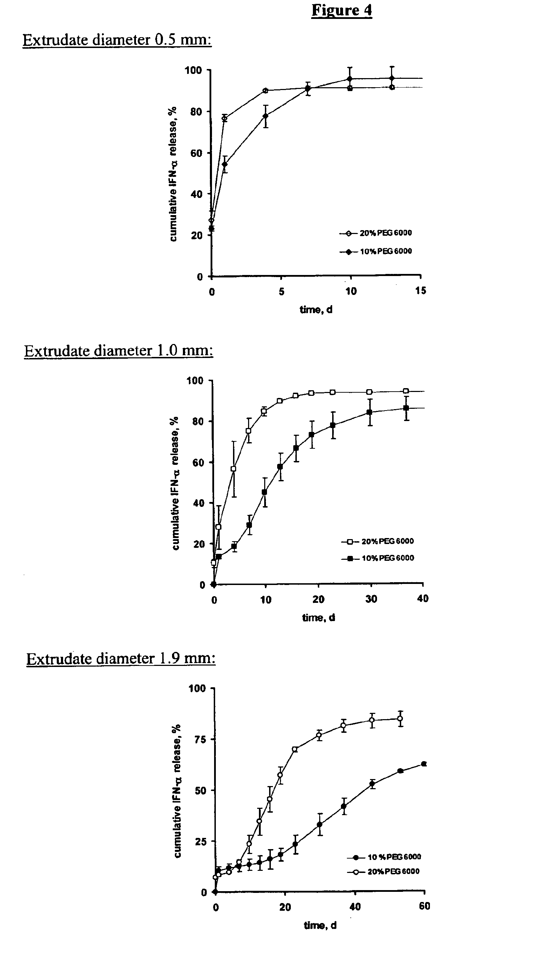

The change of the extrudate diameter resulted in changes in the kinetics of protein delivery (Figure 4). For instance, extrudates comprising 10 % PEG liberated IFN-α in a sustained manner over 13 days, when the implant diameter was 0.5 mm. In comparison, the use of extrudates with a diameter of 1.0 or 1.9 mm extended the release period up to 30 or up to 60 days, respectively. Coexistent to this prolongation of the release period, the total amount of IFN-α released differed in dependence on the implant diameter. A similar influence of the extrudate diameter was observed with implants loaded with 20 % PEG. Almost complete protein recovery was observed with extrudates of a diameter of 0.5 and 1 mm during the observation period. Furthermore, extrudates with a diameter of 1.9 mm revealed a sustained IFN-a release over 2 months. The stepwise decrease of the totally liberated IFN-α by increasing the implant diameter, indicates that the incomplete protein recovery from larger extrudates can be ascribed to the implant geometry rather than to protein aggregation within the extrudates.

Besides the possibility to adapt the release kinetics by using extrudates with different diameters the addition of various amounts of PEG is also an effective tool to modify IFN-α release (Figure 4). Increasing the amount of incorporated PEG from 10 to 20 % resulted in a more accelerated IFN-α release. For example, extrudates with a diameter of 1.0 mm comprising 10 % PEG liberated IFN-α in a sustained manner over 37 days, whereas the delivery was only retarded over 19 days when 20 % PEG were used as pore former.

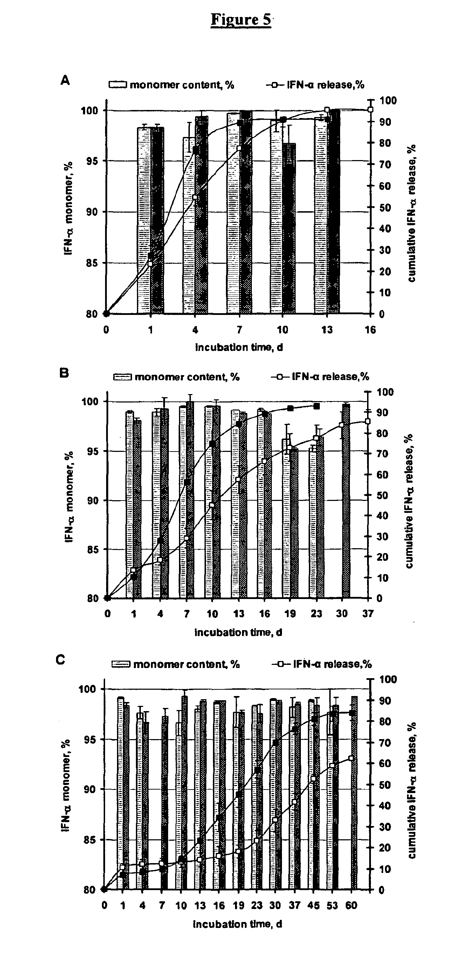

In Figure 5 the monomer content of released IFN-α versus incubation time is illustrated. Over the entire liberation period the IFN-α monomer content remained at a very high level (>95 %). Beside monomeric IFN-α only dimer specimen were detected by SE-HPLC (as described above). Mostly, the release protein resembled only between 0.5 % and 2 % dimer.

### Example 6

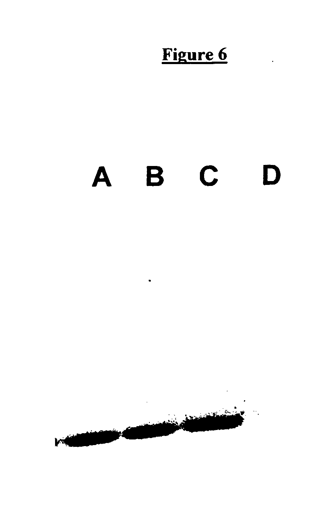

In order to exclude detrimental effects of the manufacturing procedure on lysozyme stability, lysozyme was extracted from the lipidic extrudates and analysed by SDS-PAGE (Figures 6). As shown in Figure 6, no protein degradation products were detected by Coomassie Blue staining, indicating that the developed extrusion procedure did not compromise the stability of lysozyme.

### Example 7

Lysozyme release from implants prepared by twin screw extrusion was studied in accordance to Example 5. In order to determine lysozyme stability and concentration SE-HPLC analysis was carried out. A TSKgel G3000SWXL, 7.8 mm x 30.0 mm column (Tosoh Biosep, Stuttgart, Germany) was used with a mobile phase consisting of 200 mM disodium hydrogen phosphate dihydrate (adjusted to pH 6.8 with hydrochloric acid), the flow rate was 0.4 mL/min, and lysozyme was detected spectrophotometrically (λ = 215 nm, UV 1000; Thermo Electron Cooperation, Dreieich, Germany). Calibration curves were generated with lysozyme solutions in a concentration range of 20.3 to 324.8 mg/mL.

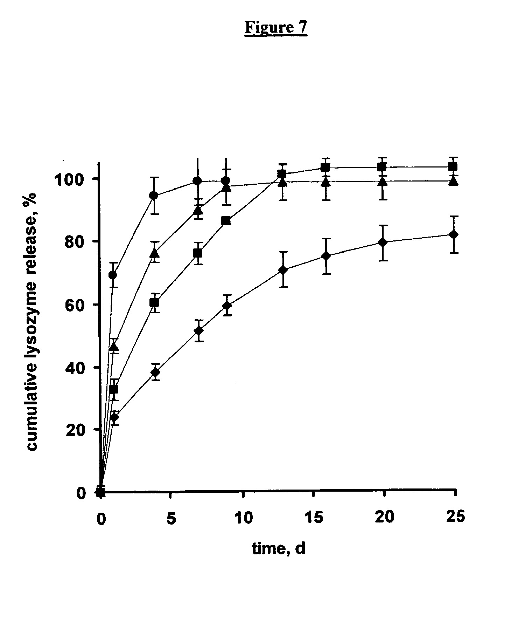

In Figure 7 the *in vitro* release kinetics of lysozyme from extrudates containing 20 % PEG are illustrated. In accordance to the delivery of IFN-α, the reduction of the implant diameter resulted in a less retarded lysozyme release. For instance, the amount of lysozyme delivered within the first 24 hours increased from 23.76 % (SD=5.33 %, n=3) to 69.51 % (SD=3.85 %, n=3) when the implant diameter was reduced from 1.9 mm to 0.5 mm.

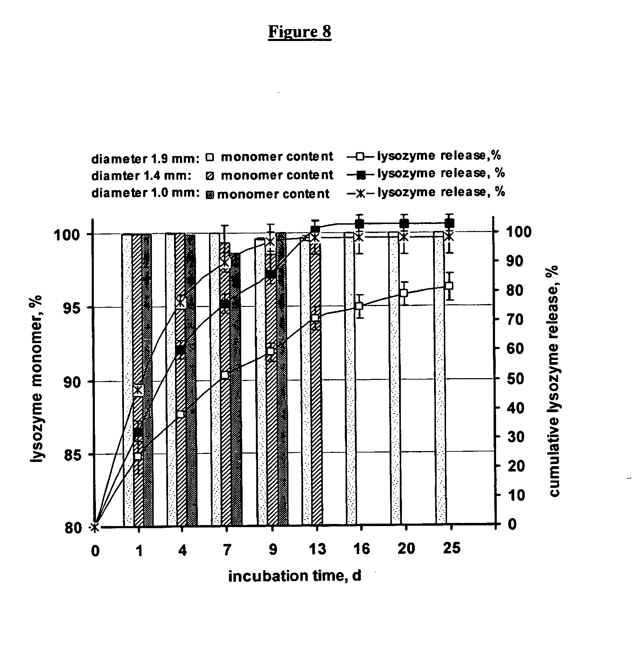

As shown in Figure 8, lysozyme was delivered almost entirely in its monomeric form (>98 %) from extrudates prepared by twin screw extrusion. Size exclusion chromatograms of lysozyme revealed a main protein peak with a retention time of 24.5 minutes.

### Example 8

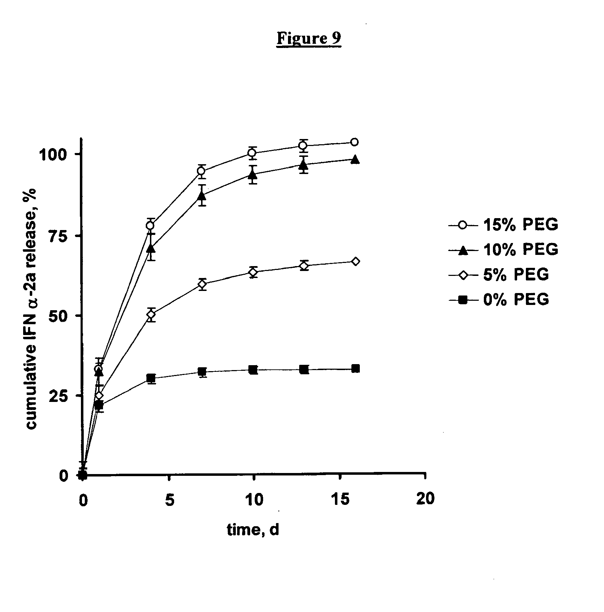

A lipid/protein mixture comprising 10 % IFN- α/HP-β-CD lyophilisate, 0 to 15 % PEG, and tristearin as matrix material was extruded by means of a ram extruder. For that purpose lipid powder was blended with lyophilised IFN- a and optionally PEG in an agate mortar. This mixture was filled into the barrel of a purpose made extruder device (Figure 9). After inserting the extruder piston a force of 3.92 kN was applied for 30 s with a hydraulic press (Maassen, Eningen, Germany). Then, the extruder was lifted on a rack and the lipidic formulation was extruded with the hydraulic press. As the extruder die was 1.2 mm, extrudates revealed a diameter of 1.2 mm. The average length of the extrudates was 15 mm.

The addition of PEG to the lipidic formulation significantly affects the *in vitro* release kinetics of IFN-α. Irrespective of the initial PEG loading, continuous protein liberation did last about 16 days.

### Example 9

The influence of different manufacturing strategies - ram extrusion, twin screw extrusion and compression - on the release of IFN-α was investigated. The lipid/protein mixture comprising 10 % IFN-α/HP-β-CD lyophilisate, 20 % PEG, and a lipidic powder blend of H12 and tristearin in a mass ratio of 1 to 4 was compressed at 19.6 kN for 30 s, extruded by means of the ram extruder or by means of twin screw extrusion as described above.

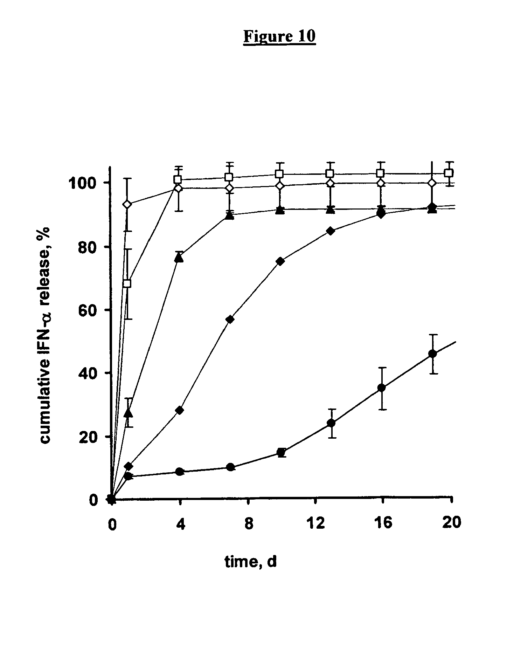

As illustrated in Figure 10, compared to the process of the invention protein liberation occurred in a significantly accelerated manner when the manufacturing of devices was accomplished by ram extrusion or by compression. It is surprising that, the smaller diameter of twin screw extruded rods resulted in a more sustained protein delivery. This suggests that twin screw extrusion according to the process of the invention *per se* causes more delayed protein release.

### Example 10

In order to investigate the distribution of the drug within the lipid matrix homogeneity studies by the admixture of methylene blue to the formulation were carried out.

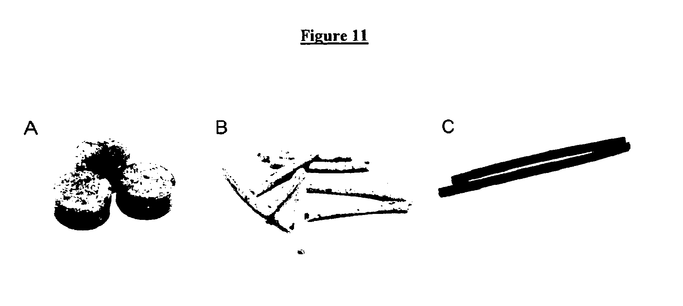

To this end, the lipidic powder comprising H12 and tristearin in a ratio of ¼ was admixed with 1 % methylene blue in mortar. The obtained powder blend was (A) compressed at 19.8 kN for 30 seconds (B) extruded with a ram extruder or (C) extruded with a twin-screw extruder as described above. As shown in Figure 11 extruded rods prepared by twin screw extrusion revealed an uniform staining. In contrast, implants of the same formulation processed by compression or ram extrusion revealed darker and brighter zones, indicating that the biological drug is more finely distributed with the lipid matrix when the manufacturing is performed by twin screw extrusion.

### Example 11

The mechanical properties of implants prepared either by compression, by ram extrusion or by twin screw extrusion were estimated with the Texture Analyser TA XT2i (Stable Micro systems, UK). A 25 mm cylinder probe was used, and the implants were placed centrally under the probe. The standardised test program "Failure behaviour of tablets due to diametrical compression using a cylinder probe" (settings: pre-test speed 2 mm/s, test speed 0.03 mm/s and post-test speed 10 mm/s) was applied. From the obtained force versus time plot the maximum force value was used as the longitudinal tensile strength.

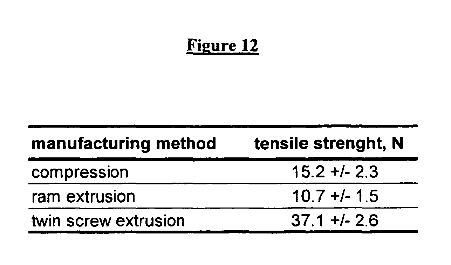

The manufacturing by twin screw extrusion significantly improved the mechanical stabilities of the implants (Figure 12). This more compact matrix structure might be beneficial with regard to administration, transport and handling of the extruded device according to the invention.

### Example 12

### Adjustment of in-vitro release kinetics by temperature treatment

A lipid powder comprising:

| | |
|---|---|
| H12 | 24% |
| D118 | 56% |
| PEG 6000 | 17.5% |
| Lysozyme | 2.5% |

was prepared by grinding in a mortar. Extrusion was performed using a twin screw extruder (MiniLab Micro Rheology Compounder, Thermo Haake GmbH Karlsruhe, Germany). The extruder was heated to 40 °C prior filling. The rotation speed of the screws was fixed at 40 rpm. Then the extruder was manually filled with the lipid blend. Extrusion was performed with closed bypass channel to allow a direct extrusion without circulating.

In-vitro release studies were carried out in phosphate buffer saline pH 7.4 (PBS, 1.44 g/L Na₂HPO₄ * 2H₂O, 0.2 g/L KH₂PO₄, 8.0 g/L NaCl, 0.2 g/L KCl, 0.5 g/L NaN₃) at 40 rpm at 20 and at 37°C, respectively. The delivered amount of lysozyme was determined by SE-HPLC using Superose 12 column (GE Healthcare) with a mobile phase consisting of 200 mM disodium hydrogen phosphate dihydrate (adjusted to pH 6.8 with hydrochloric acid), the flow rate was 0.62 mL/min, and lysozyme was detected spectrophotometrically.

After extrusion differential scanning calorimetry (DSC) measurements revealed a depression in the melting points. The low melting lipid component already melts at 29.82 °C (n=3, SD=0.23) after extrusion whereas H12 bulk material melts at 35.6 °C (n=3, SD=0.7). This means that a fraction of the extrudates melts during in-vitro incubation at 37°C. However, regardless of melting the implant systems appeared macroscopic stable.

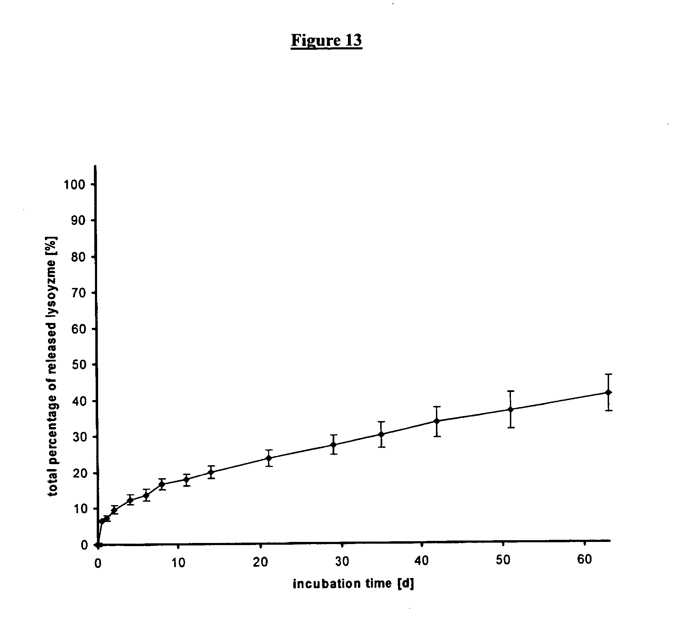

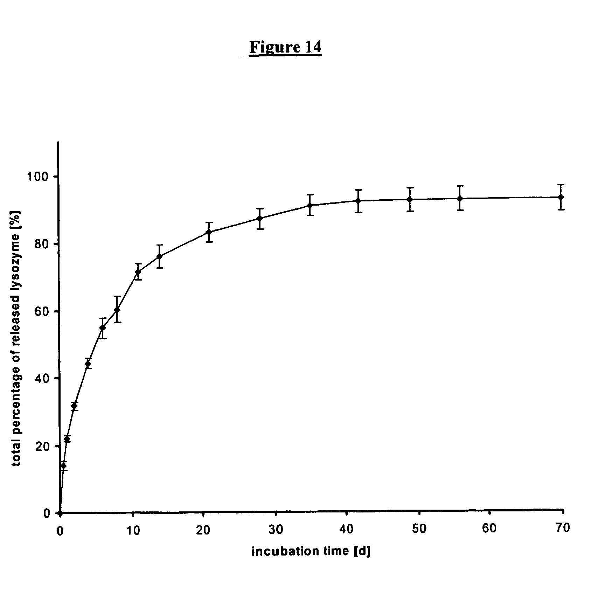

Compared to the incubation at 20°C (Figure 13) the melting at 37°C (Figure 14) resulted in an accelerated protein release. This shows that the extruded rod-shaped devices of the invention can be used as a delivery system that allows the temperature-dependent modulate release of the biological substance at the site of action (temperature variations can be adjusted by a separate heat treatment or in combination with certain diseases (such as inflammatory reactions) or treatments (such as laser therapy)). Without wishing to be bound by any theory, it is believed that these observations are explained in terms of changes in lipid structure and conformation that happen in the implant.

### Example 13

### Effect of different excipients on solubility and/or degradation

### 1. Excipients used as a precipitation agent

The following excipient protein combinations can be used in the developed implant system to reduce the solubility of the protein within the implant pores. Due to in-situ protein precipitation during release the protein concentration within the small-sized pore volume is reduced which in turn accounts for reduced burst effects and more sustained protein delivery (S. Herrmann, S. Mohl, F. Siepmann, J. Siepmann, G. Winter, New insight into the role of polyethylene glycol acting as protein release modifier in lipidic implants, Pharm. Res., 24 (2007) 1527-1537).

### 1.1. Interferon

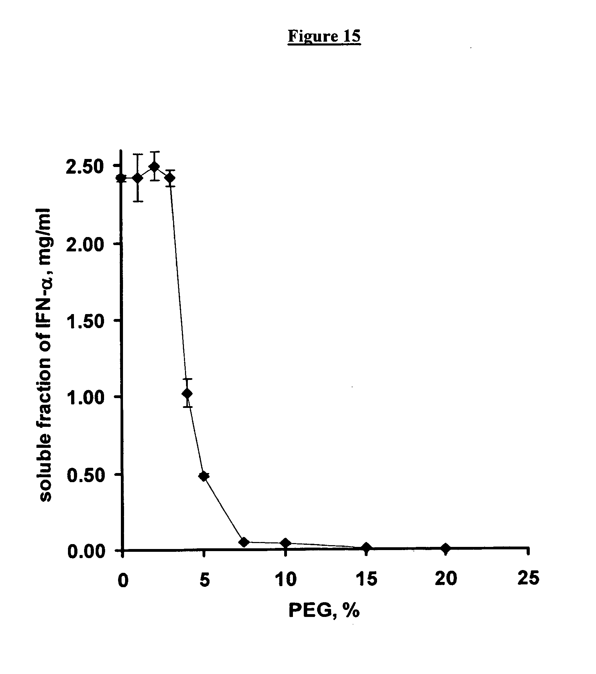

Solutions of poly(ethylene glycol) 6000 with a concentration of 2-40 % (wt/vol) in PBS 7.4 were prepared. These solutions were mixed in a ratio of 1:1 with IFN-α bulk solutions (initial concentration of 4.9 mg/mL 7.4). Afterwards, the samples were equilibrated for 2 h at 37°C, 40 rpm (Certomat IS). Subsequently, precipitated protein was separated by centrifugation at 5000 rpm (5 °C, 5 min, 4K15 laboratory centrifuge; Sigma, Osterode, Germany) and the residual IFN-α concentration in the supernatant was determined. Thus, solubility is referred to the solute concentration of the supernatant in equilibrium with the precipitated phase. Protein concentration was determined by reversed phase chromatography (see Figure 15).

### 1.2. Lysozym

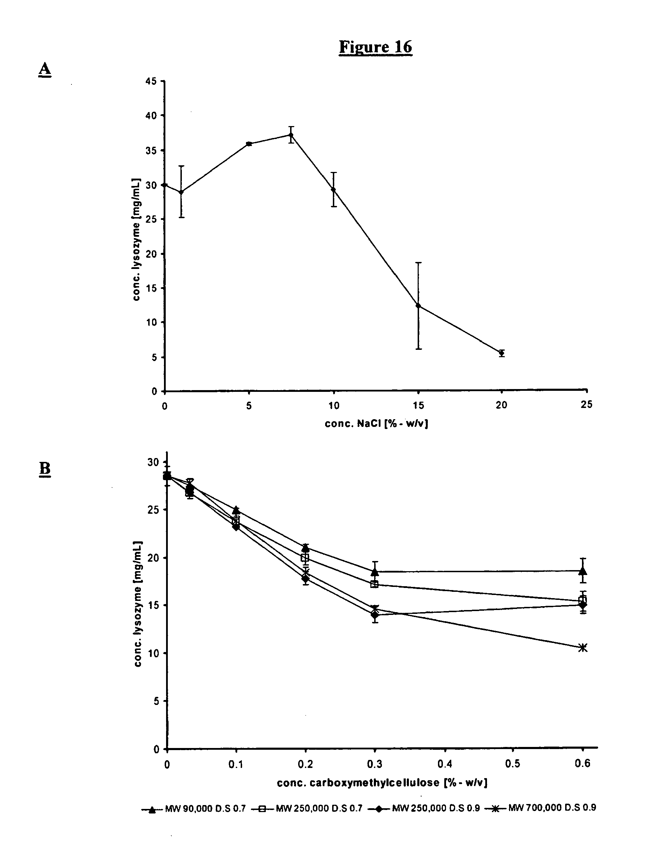

Different concentrations of NaCl (0-40% wt/vol) or carboxymethylcellulose (0-1.2% wt/vol) were prepared in PBS 7.4. These solutions were mixed in a ratio of 1:1 with lysozym bulk solutions (initial concentration of 60 mg/mL 7.4). Afterwards, the samples were equilibrated for 2 h at 37°C, 40 rpm (Certomat IS). Subsequently, precipitated protein was separated by centrifugation at 5000 rpm (5°C, 5 min, 4K15 laboratory centrifuge; Sigma, Osterode, Germany) and the residual lysozyme concentration in the supernatant was determined by UV absorbance at 280 nm (see Figures 16a and 16b).

### 1.3. Monoclonal antibody (IgG1)

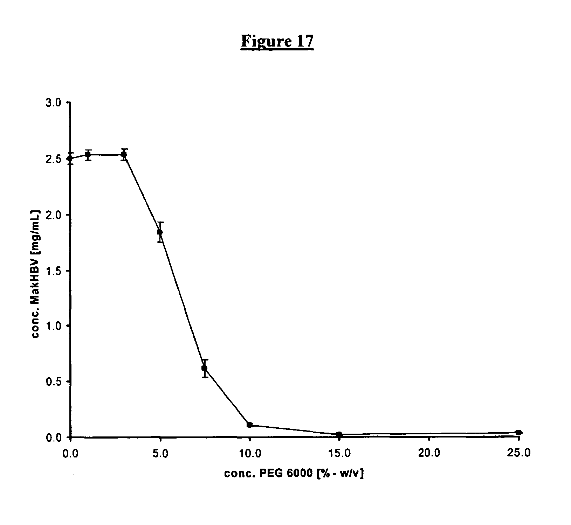

Solutions of poly(ethylene glycol) 6000 with a concentration of 2-50 % (wt/vol) in PBS 7.4 were prepared. These solutions were mixed in a ratio of 1:1 with IgG1 bulk solutions (initial concentration of 5.0 mg/mL 7.4). Afterwards, the samples were equilibrated for 2 h at 37°C, 40 rpm (Certomat IS). Subsequently, precipitated protein was separated by centrifugation at 5000 rpm (5 °C, 5 min, 4K15 laboratory centrifuge; Sigma, Osterode, Germany) and the IgG concentration in the supernatant was determined by UV absorbance at 280 nm (see Figure 17).

### 2. Excipients to modify the erosion behaviour of the implant system

Beside the addition of lipid qualities that trigger the erosion of the implant system swellable excipients such as carboxymethylcellulose can accelerate the erosion of the implant system. Such an effect may or may not affect the release kinetics of the extrudate.

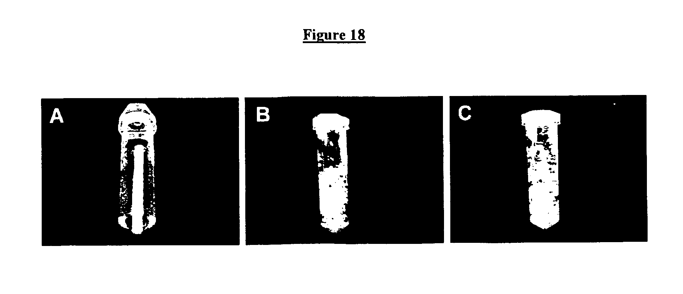

Extrudates containing various amounts 2% of carboxymethylcellulose (MW 700,000, D.S. 0.9) were prepared by twin-screw extrusion (40°C, 40 rpm). The extrudates further compromised: 24% H12, 64 % D118 and 18 % PEG. As shown in picture A to C of Figure 18 these extrudates collapsed within 24 hours.

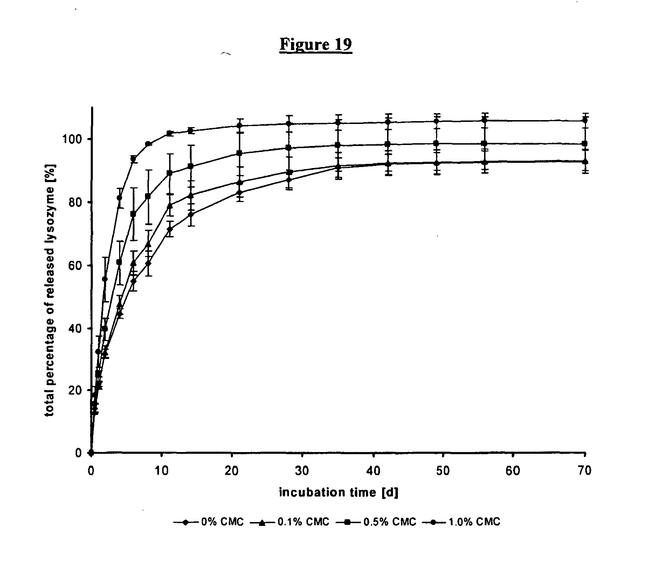

The in-vitro release of lysozyme at 37°C from extrudates containing 0, 0.1%, 0.5%, and 1% carboxymethylcellulose was investigated. As it can be seen in Figure 19 the addition of carboxymethylcellulose to the implant formulation resulted in accelerated protein release. Even though the implants remained stable it is highly likely that the enhanced degradation shown in figure 18 for a higher carboxymethylcellulose concentration accounts for this observation. Both effects, implant swelling and increased water penetration, which facilitate the degradation would result in increased release rates when carboxymethylcellulose is added.

## Claims

1. An extruded rod-shaped device for sustained delivery of biological substances, obtainable by a process comprising
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the preparation of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

2. The device according to claim 1, wherein the melting point of the high melting lipid or lipoid component is at least 10°C higher than the melting point of the low melting lipid or lipoid component.

3. The device according to claim 1 or 2, wherein the melting point of the high melting lipid or lipoid component is above 50°C and/or the melting point of the low melting lipid or lipoid component is below 50°C.

4. The device of according to any of claims 1 to 3, wherein both the high melting lipid or lipoid component and the low melting lipid or lipoid component are selected from the class of fatty acid mono-, di- and/or triglycerides, and salts and derivatives thereof.

5. The device according to any of claims 1 to 4, wherein the at least one biological substance is selected from the group consisting of proteins, polypeptides, peptides and nucleic acids, and salts and derivatives thereof, or is a virus-like particle.

6. The device according to any of claims 1 to 5, wherein both the high melting lipid or lipoid component and the low melting lipid or lipoid component have a stable lipid modification after extrusion.

7. The device according to any of claims 1 to 6, wherein the at least one biological substance is delivered over a period of at least one week.

8. A method of producing a rod-shaped device for sustained delivery of a biological substance, comprising
(a) providing a preparation which comprises at least 50% per weight of a lipoid composition and at least one biological substance, the lipoid composition comprising a high melting lipid or lipoid component and a low melting lipid or lipoid component, wherein the melting point of the low melting lipid or lipoid component is lower than the melting point of the high melting lipid or lipoid component;
(b) extruding the composition of (a) at a temperature, which is at or above the melting point of the low melting lipid or lipoid component but below the melting point of the high melting lipid or lipoid component, in a screw type extruder; and
(c) obtaining the extruded rod-shaped device from the extrudate of (b).

9. The method according to claim 8, wherein the preparation further comprises at least one excipient which (i) modifies the release of the at least one biologically active substance from the implantable device and/or (ii) modifies the biodegradation of the lipids or lipoid components of the implantable device and/or (iii) stabilises the biological substance and/or (iv) modifies the solubility of the biological substance and/or (v) features slow dissolution behavior, wherein the at least one excipient preferably is selected from the group consisting of a hydrophilic polymer, a sugar, a polyol, a surfactant and a water-soluble salt.

10. The method according to claim 9, wherein the hydrophilic polymer is selected from the group consisting of polyethylene glycol, polyvinylpyrrolidone, polyvinylalcohol, dextran, dextran sulfate, chondroitin sulfate, dermatan sulfate, heparan sulfate, keratan sulfate, hyaluronic acid, chitosan, albumin, fibrin, cyclodextrin and mixtures thereof.

11. The method according to any of claims 8 to 10, wherein extrusion is carried out at a temperature which is between 1°C and 25°C above the melting point of the low melting lipid or lipoid component, preferably between 1°C and 20°C above the melting point of the low melting lipid or lipoid component, and more preferably between 1°C and 10°C above the melting point of the low melting lipid or lipoid component.

12. The method according to any of claims 8 to 11, wherein extrusion is carried out at a temperature which is between 40°C and 60°C.

13. The method according to any of claims 8 to 12, wherein the extruder is equipped with at least one pair of fully intermeshing, co-rotating, extruder elements, and preferably is a twin screw extruder.

14. The method according to any of claims 8 to 13, wherein the production process of the extruded rod-shaped device is a single-step process.

15. Use of the extruded rod-shaped device according to any of claims 1 to 7, or produced by the method according to any one of claims 8 to 14, as a parenteral delivery system for sustained delivery of biological substances.

## Patentansprüche

1. Ein extrudiertes stäbchenförmiges Mittel zur anhaltenden Freisetzung von biologischen Substanzen, erhältlich durch ein Verfahren umfassend
(a) Bereitstellen einer Zubereitung, welche mindestens 50 Gew.-% von einer lipoiden Zusammensetzung und mindestens eine biologische Substanz umfasst, die lipoide Zusammensetzung umfassend eine hochschmelzende Lipid- oder Lipoid-Komponente und eine niedrigschmelzende Lipid- oder Lipoid-Komponente, wobei der Schmelzpunkt der niedrigschmelzenden Lipid- oder Lipoid-Komponente niedriger als der Schmelzpunkt der hochschmelzenden Lipid- oder Lipoid-Komponente ist;
(b) Extrudieren der Zubereitung aus (a) bei einer Temperatur, welche bei oder über dem Schmelzpunkt der niedrigschmelzenden Lipid- oder Lipoid-Komponente ist, aber unter dem Schmelzpunkt der hochschmelzenden Lipid- oder Lipoid-Komponente ist, in einem Schneckenextruder; und
(c) Erhalten der extrudierten stäbchenförmigen Mittel aus dem Extrudat aus (b).

2. Das Mittel gemäß Anspruch 1, wobei der Schmelzpunkt der hochschmelzenden Lipid- oder Lipoid-Komponente mindestens 10 °C höher ist als der Schmelzpunkt der niedrigschmelzenden Lipid- oder Lipoid-Komponente.

3. Das Mittel gemäß Anspruch 1 oder 2, wobei der Schmelzpunkt der hoch schmelzenden Lipid- oder Lipoid-Komponente über 50 °C ist und/oder der Schmelzpunkt der niedrigschmelzenden Lipid- oder Lipoid-Komponente niedriger als 50 °C ist.

4. Das Mittel gemäß einem der Ansprüche 1 bis 3, wobei sowohl die hochschmelzende Lipid- oder Lipoid-Komponente als auch die niedrigschmelzende Lipid- oder Lipoid-Komponente ausgewählt sind aus der Klasse der Fettsäure-Mono-, Di- und/oder Triglyceride und Salze und Derivate davon.

5. Das Mittel gemäß einem der Ansprüche 1 bis 4, wobei die mindestens eine biologische Substanz ausgewählt ist aus der Gruppe bestehend aus Proteinen, Polypeptiden, Peptiden und Nukleinsäuren, sowie Salzen und Derivaten davon, oder es ist ein Virus-ähnlicher Partikel.

6. Das Mittel gemäß einem der Ansprüche 1 bis 5, wobei sowohl die hochschmelzende Lipid- oder Lipoid-Komponente wie auch die niedrigschmelzende Lipid- oder Lipoid-Komponente eine stabile Lipid-Modifikation nach der Extrusion haben.

7. Das Mittel gemäß einem der Ansprüche 1 bis 6, wobei die mindestens eine biologische Substanz über einen Zeitraum von mindestens einer Woche abgegeben wird.

8. Ein Verfahren zur Herstellung eines stäbchenförmigen Mittels zur anhaltenden Freisetzung von einer biologischen Substanz, umfassend
(a) Bereitstellen einer Zubereitung, welche mindestens 50 Gew.-% von einer lipoiden Zusammensetzung und mindestens eine biologische Substanz umfasst, die lipoide Zusammensetzung umfassend eine hochschmelzende Lipid- oder Lipoid-Komponente und eine niedrigschmelzende Lipid- oder Lipoid-Komponente, wobei der Schmelzpunkt der niedrigschmelzenden Lipid- oder Lipoid-Komponente niedriger als der Schmelzpunkt der hochschmelzenden Lipid- oder Lipoid-Komponente ist;
(b) Extrudieren der Zubereitung von (a) bei einer Temperatur, welche bei oder über dem Schmelzpunkt der niedrigschmelzenden Lipid- oder Lipoid-Komponente ist, aber unter dem Schmelzpunkt der hochschmelzenden Lipid- oder Lipoid-Komponente ist, in einem Schneckenextruder; und
(c) Erhalten der extrudierten stäbchenförmigen Mittel aus dem Extrudat aus (b).

9. Das Verfahren gemäß Anspruch 8, wobei die Zubereitung ferner mindestens einen Hilfsstoff umfasst, welcher (i) die Freisetzung von der mindestens einen biologisch aktiven Substanz von dem implantierbaren Mittel modifiziert, und/oder (ii) den biologischen Abbau der Lipid- oder Lipoid-Komponenten von dem implantierbaren Mittel modifiziert, und/oder (iii) die biologische Substanz stabilisiert, und/oder (iv) die Löslichkeit von der biologischen Substanz modifiziert, und/oder (v) langsames Auflösungsverhalten aufweist, wobei der mindestens eine Hilfsstoff bevorzugt ausgewählt ist aus der Gruppe bestehend aus einem hydrophilen Polymer, einem Zucker, einem Polyol, einem Tensid und einem wasserlöslichen Salz.

10. Das Verfahren gemäß Anspruch 9, wobei das hydrophile Polymer ausgewählt ist aus der Gruppe bestehend aus Polyethylenglykol, Polyvinylpyrrolidon, Polyvinylalkohol, Dextran, Dextransulfat, Chondroitinsulfat, Dermatansulfat, Heparansulfat, Keratansulfat, Hyaluronsäure, Chitosan, Albumin, Fibrin, Cyclodextrin und Gemische davon.

11. Das Verfahren gemäß einem der Ansprüche 8 bis 10, wobei die Extrusion bei einer Temperatur durchgeführt wird, welche zwischen 1 °C und 25°C über dem Schmelzpunkt der niedrigschmelzenden Lipid- oder Lipoid-Komponente ist, bevorzugt zwischen 1°C und 20°C über dem Schmelzpunkt der niedrigschmelzenden Lipid- oder Lipoid-Komponente, und mehr bevorzugt zwischen 1 °C und 10°C über dem Schmelzpunkt der niedrigschmelzenden Lipid- oder Lipoid-Komponente.

12. Das Verfahren gemäß einem der Ansprüche 8 bis 11, wobei die Extrusion bei einer Temperatur durchgeführt wird, welche zwischen 40°C und 60°C ist.

13. Das Verfahren gemäß einem der Ansprüche 8 bis 12, wobei der Extruder ausgestattet ist mit mindestens einem Paar von voll ineinander greifenden, gleichlaufenden Extruder-Elementen und bevorzugt ein Zweischneckenextruder ist.

14. Das Verfahren gemäß einem der Ansprüche 8 bis 13, wobei der Herstellungsprozess der extrudierten stäbchenförmigen Mittel ein Einzelschrittverfahren ist.

15. Verwendung der extrudierten stäbchenförmigen Mittel gemäß einem der Ansprüche 1 bis 7, oder hergestellt durch das Verfahren gemäß einem der Ansprüche 8 bis 14, als ein parenterales Verabreichungssystem zur anhaltenden Freisetzung von biologischen Substanzen.

## Revendications

1. Dispositif extrudé en forme de baguette pour l'administration prolongée de substances biologiques, pouvant être obtenu par un procédé comprenant
(a) la fourniture d'une préparation qui comprend au moins 50% en poids d'une composition lipoïde et au moins une substance biologique, la composition lipoïde comprenant un constituant lipide ou lipoïde de point de fusion élevé et un constituant lipide ou lipoïde de faible point de fusion, dans lequel le point de fusion du constituant lipide ou lipoïde de faible point de fusion est inférieur au point de fusion du constituant lipide ou lipoïde de point de fusion élevé ;
(b) l'extrusion de la préparation de (a) à une température qui est égale ou supérieure au point de fusion du constituant lipide ou lipoïde de faible point de fusion mais inférieure au point de fusion du constituant lipide ou lipoïde de point de fusion élevé, dans un extrudeuse à vis; et
(c) l'obtention du dispositif extrudé en forme de baguette à partir de l'extrudat de (b).

2. Le dispositif selon la revendication 1, dans lequel le point de fusion du constituant lipide ou lipoïde de point de fusion élevé est d'au moins 10°C supérieur au point de fusion du constituant lipide ou lipoïde de faible point de fusion.

3. Le dispositif selon la revendication 1 ou 2, dans lequel le point de fusion du constituant lipide ou lipoïde de point de fusion élevé est supérieur à 50°C et/ou le point de fusion du constituant lipide ou lipoïde de faible point de fusion est inférieur à 50°C.

4. Le dispositif selon l'une quelconque des revendications 1 à 3, dans lequel à la fois le constituant lipide ou lipoïde de point de fusion élevé et le constituant lipide ou lipoïde de faible point de fusion sont sélectionnés dans la classe des mono, di et/ou triglycérides d'acide gras, et leurs sels et dérivés.

5. Le dispositif selon l'une quelconque des revendications 1 à 4, dans lequel l'au moins une substance biologique est sélectionnée dans le groupe constitué par les protéines, les polypeptides, les peptides et les acides nucléiques, et leurs sels et dérivés, ou est une particule de type viral.

6. Le dispositif selon l'une quelconque des revendications 1 à 5, dans lequel à la fois le constituant lipide ou lipoïde de point de fusion élevé et le constituant lipide ou lipoïde de faible point de fusion présentent une modification lipidique stable après extrusion.

7. Le dispositif selon l'une quelconque des revendications 1 à 6, dans lequel l'au moins une substance biologique est administrée durant une période d'au moins une semaine.

8. Procédé de production d'un dispositif en forme de baguette pour l'administration prolongée d'une substance biologique, comprenant
(a) la fourniture d'une préparation qui comprend au moins 50% en poids d'une composition lipoïde et au moins une substance biologique, la composition lipoïde comprenant un constituant lipide ou lipoïde de point de fusion élevé et un constituant lipide ou lipoïde de faible point de fusion, dans lequel le point de fusion du constituant lipide ou lipoïde de faible point de fusion est inférieur au point de fusion du constituant lipide ou lipoïde de point de fusion élevé ;
(b) l'extrusion de la composition de (a) à une température qui est égale ou supérieure au point de fusion du constituant lipide ou lipoïde de faible point de fusion mais inférieure au point de fusion du constituant lipide ou lipoïde de point de fusion élevé, dans un extrudeuse à vis ; et
(c) l'obtention du dispositif extrudé en forme de baguette à partir de l'extrudat de (b).

9. Le procédé selon la revendication 8, dans lequel la préparation comprend en outre au moins un excipient qui (i) modifie la libération de l'au moins une substance biologiquement active à partir du dispositif implantable et/ou (ii) modifie la biodégradation des lipides ou constituants lipoïdes du dispositif implantable et/ou (iii) stabilise la substance biologique et/ou (iv) modifie la solubilité de la substance biologique et/ou (v) présente un comportement de dissolution lente, dans lequel l'au moins un excipient est de préférence sélectionné dans le groupe constitué par un polymère hydrophile, un sucre, un polyol, un surfactant et un sel hydrosoluble.

10. Le procédé selon la revendication 9, dans lequel le polymère hydrophile est sélectionné dans le groupe constitué par le glycol polyéthylénique, la polyvinylpyrrolidone, le polyvinylalcool, le dextrane, le sulfate de dextrane, le sulfate de chondroïtine, le sulfate de dermatane, le sulfate d'héparane, le sulfate de kératane, l'acide hyaluronique, la chitosane, l'albumine, la fibrine, la cyclodextrine et leurs mélanges.

11. Le procédé selon l'une quelconque des revendications 8 à 10, dans lequel l'extrusion est effectuée à une température qui est entre 1°C et 25°C supérieure au point de fusion du constituant lipide ou lipoïde de faible point de fusion, de préférence entre 1 °C et 20°C supérieure au point de fusion du constituant lipide ou lipoïde de faible point de fusion, et plus préférentiellement entre 1 °C et 10°C supérieure au point de fusion du constituant lipide ou lipoïde de faible point de fusion.

12. Le procédé selon l'une quelconque des revendications 8 à 11, dans lequel l'extrusion est effectuée à une température qui est comprise entre 40°C et 60°C.

13. Le procédé selon l'une quelconque des revendications 8 à 12, dans lequel l'extrudeuse est équipée d'au moins une paire d'éléments d'extrudeuse en co-rotation entièrement engrenants, et est de préférence une extrudeuse à double vis.

14. Le procédé selon l'une quelconque des revendications 8 à 13, dans lequel le procédé de production du dispositif extrudé en forme de baguette est un procédé en une étape.

15. Utilisation du dispositif extrudé en forme de baguette selon l'une quelconque des revendications 1 à 7, ou produit par le procédé selon l'une quelconque des revendications 8 à 14, comme système d'administration parentérale pour l'administration prolongée de substances biologiques.
